# EUROPEAN PATENT APPLICATION

(11) **EP 4 666 996 A1**
(43) Date of publication of application: **24.12.2025**
(21) Application number: 24183625.3
(22) Date of filing: 21.06.2024
(51) Int. Cl.: A61K 8/64, A61Q 19/08

(54) **COMPOSITION CONTAINING PEPTIDES OF VEGETABLE ORIGIN**

(71) Applicant: Quiris Healthcare GmbH & Co. KG, 33334 Gütersloh (DE); A.Costantino & C. S.p.A., 10083 Favria (IT)
(72) Inventor: Kattenstroth, Jan-Christoph, 33334 Gütersloh (DE); Rivardo, Fabrizio, 13878 Candelo (IT); Scavarda, Camilla, 10010 Rueglio (IT)
(74) Representative: Ter Meer Steinmeister & Partner

(57) **Abstract**

The present invention relates to a composition comprising at least a first peptide of a first vegetable origin having bioactive properties. In particular, the at least first peptide of a first vegetable origin has skin physiology and/or morphology modifying and/or anti-aging properties. This composition can be used as a cosmetic, a cosmeceutical, a dietary or food supplements, food for special medical purposes, or as a nutraceutical. The present invention is also directed to peptides having SEQ-ID:1 to SEQ-ID:62 which are bioactive.

## Description

### FIELD OF THE INVENTION

The present invention relates to a composition comprising at least a first peptide of a first vegetable origin having bioactive properties. In particular, the at least first peptide of a first vegetable origin has skin physiology and/or morphology modifying and/or anti-aging properties. This composition can be used as a cosmetic, a cosmeceutical, a dietary or food supplements, food for special medical purposes, or as a nutraceutical. The present invention is also directed to specific peptides having bioactive properties.

### BACKGROUND OF THE INVENTION

The skin is the largest organ in the body and the first barrier to the external insults and its integrity and health is the base of our survival. Healthy skin regulates body temperature, controlling the transfer of heat out of the body. It assures protection from UV radiation, water loss as well as physical and chemical injury. The skin is the first barrier against microorganisms. Our perception of beauty is strictly related to the skin health status: the quality and condition of the skin greatly contributes to the perception of health, wellness, youth, and beauty.

Skin aging is characterized by changes in its structural, cellular, and molecular components in both the epidermis and dermis. Dermal aging is distinguished by reduced dermal thickness, increased wrinkles, and a sagging appearance. Due to intrinsic or extrinsic factors, accumulation of excessive reactive oxygen species (ROS) triggers a series of aging events, such as loss of cell identity, accumulation of senescent fibroblasts, elastin and collagen production reduction concomitant to increased degradation (see Zhang, J., Yu, H., Man, M. & Hu, L. Aging in the dermis: Fibroblast senescence and its significance. Aging Cell 23, e14054 (2024)).

Constantly exposed to external insults, the skin undergoes significant changes through our lifetime that differentiate the skin of a child from that of an older adult. These changes are caused by a combination of intrinsic aging and extrinsic aging induced by environmental factors, including air pollution, poor nutrition, smoking, and ultraviolet (UV) light. Both epidermal keratinocyte and dermal fibroblast senescence contribute to the skin aging (see Gruber, F., Kremslehner, C., Eckhart, L. & Tschachler, E. Cell aging and cellular senescence in skin aging - Recent advances in fibroblast and keratinocyte biology. Exp. Gerontol. 130, 110780 (2020) and Fitsiou, E., Pulido, T., Campisi, J., Alimirah, F. & Demaria, M. Cellular senescence and the senescence-associated secretory phenotype as drivers of skin photoaging. 141, 1119-1126).

Skin ageing or fatigue is often directly visible in many areas. Skin ageing on the face is particularly noticeable, but other areas of the human body are also affected by ageing and its signs. In addition to the cells' limited ability to regenerate, the reduction of the subcutaneous fat layer plays an important role. The stabilizing collagen fibres of the skin also lose strength with increasing age and are even partially broken down. The collagen content is directly related to the density, elasticity, firmness and volume of the skin. With age, the skin's ability to replenish its collagen stores decreases and the skin ages. If the collagen deficiency in the deeper layers of the skin is compensated the healthy skin structure and function can be restored.

Topical applications of cosmetics such as creams or lotions can only compensate for the deficiency in the short term. It has already been shown that an oral supply of liquid collagen peptides can slow down and even reverse the age-related, degenerative processes in the skin's cell metabolism. The size of these collagen peptides is important for their bioavailability and therefore for their effectiveness. Only smaller peptides with a molecular weight of approx. 300 to 6,000 Da are effectively absorbed by the body and can sufficiently stimulate collagen synthesis in the extracellular matrix (see Proksch et al., Skin Pharmacol Physiol, 2014, 27, 47-55). Corresponding collagen peptides, which can be applied orally, are disclosed, for example, in WO 2012/065782 A.

The precise composition of the amino acids in the collagen peptides is of decisive importance for determining their biological activity. Notably, a high similarity to human collagen is essential to maximize their efficacy (Bolke et al, Nutrients, 2019). For example, studies have shown that special dipeptides significantly stimulate cell proliferation and hyaluronic acid synthesis in cultured human skin fibroblasts. This activity is mediated by the upregulation of mRNA levels of hyaluronan synthase 2 (HAS2), which is essential for the modulation of extracellular matrix and cell activity. The precise amino acid sequence and structure of these peptides are critical for their integration and functionality in human biological systems, making them extremely useful for applications in skin regeneration (Ohara et al, Journal of Dermatology, 2010).

With an increasing number of people avoiding using animal products or products containing ingredients of animal origin, there is a need for food, dietary or food supplements, food for special medical purposes, nutraceutical, cosmeceutical and cosmetic products containing ingredients of vegetable origin, in particular bioactive ingredients.

It is thus an object of the present invention to provide a composition suitable for food, dietary or food supplements, food for special medical purposes, nutraceutical, cosmeceutical and cosmetic products containing bioactive ingredients having a vegetable origin. It is a further object of the present invention to provide a composition suitable for food, dietary or food supplements, food for special medical purposes, nutraceutical, cosmeceutical and cosmetic products containing bioactive ingredients having a vegetable origin, in particular having skin physiology and/or morphology modifying and/or anti-aging properties.

### SUMMARY OF THE INVENTION

It has been surprisingly found that peptides of vegetable origin have specific bioactive properties. In particular, some peptides have skin physiology and/or morphology modifying and/or anti-aging properties, such as antioxidant activity, modulation of collagen and elastin protein levels as well as modulation of collagen and elastin secretion. In addition, they surprisingly also have anti-inflammatory properties. These properties make them ideal for use in food, dietary or food supplements, food for special medical purposes, nutraceutical, cosmeceutical and cosmetic products, particularly for restoring healthy skin structure and function. It has been further surprisingly found that these peptides are absorbed without being digested in the gastrointestinal tract, so that they can be administered orally, e.g. in liquid compositions.

The properties of the peptides of the present invention also make them ideal for replacing collagen peptides of animal origin with peptides of plant origin in foods, dietary or nutritional supplements, foods for special medical purposes, nutraceutical, cosmeceutical and cosmetic products.

In a first aspect, the present invention thus provides a composition comprising at least a first peptide of a first vegetable origin having skin physiology and/or morphology modifying and/or anti-aging properties.

In an embodiment of the invention, the composition further contains at least a second peptide of a second vegetable origin having in skin physiology and/or morphology modifying and/or anti-aging properties.

Preferably, the at least first peptide and the at least second peptide are of different vegetable origin.

The at least first peptide of a first vegetable origin and - if present - the at least second peptide of a second vegetable origin are bioactive. In particular, they have skin physiology and/or morphology modifying and/or anti-aging properties.

In the present invention, the at least first peptide and/or the at least second peptide can be a hydrolyzed peptide derived from, obtained from or contained in a hydrolysate.

Due to the properties of the at least first peptide and optionally also of the at least second peptide, the composition of the present invention can be used for several applications, such as in food, dietary or food supplements, food for special medical purposes, nutraceutical, cosmeceutical and cosmetic products.

In a second aspect of the invention, a peptide being one of the peptides of SEQ-ID:1 to SEQ-ID:62 or any mixture of two or more of these peptides is claimed.

The claimed peptides were surprisingly found to have bioactive properties. In particular, the claimed peptides have skin physiology and/or morphology modifying and/or anti-aging properties, such as antioxidant activity, modulation of collagen and elastin protein levels as well as modulation of collagen and elastin secretion. They further also have anti-inflammatory properties.

### BRIEF DESCRIPTION OF THE DRAWINGS

- Fig. 1: shows the results of the ABTS assay with pea and rice peptides of Example 1 alongside results of ABTS assay with collagen and ascorbic acid.
- Fig. 2: shows the results of the FRAP assay with pea and rice peptides of Example 2 alongside results of ABTS assay with collagen and ascorbic acid.
- Fig. 3: shows the results of the viability assay on BJ-5ta cells with pea and rice peptides of Example 3.
- Fig. 4: shows the results of the modulation of ROS levels with pea and rice peptides of Example 4.
- Fig. 5: shows the results of the modulation of lipid peroxidation with pea and rice peptides of Example 5.
- Fig. 6: shows the evaluation of the protein levels modulation of collagen and elastin with pea and rice peptides of Example 6.
- Fig. 7: shows the evaluation of the protein levels modulation of phospho(Ser473)-Akt and phospho(Thr172)-AMPK with pea and rice peptides of Example 7.
- Fig. 8: shows the evaluation of the ECM stability (collagen and elastin secretion) with pea and rice peptides of Example 8.
- Fig. 9: shows the evaluation of the ECM degradation (MMP-2 and MMp-9 secretion) with pea and rice peptides of Example 9.
- Fig. 10: shows the evaluation of the protein levels modulation of NF-kB with pea and rice peptides of Example 10.
- Fig. 11: shows the evaluation of the anti-inflammatory and pro-inflammatory cytokines secretion with pea and rice peptides of Example 11.
- Fig. 12: shows the MS-spectra of pea hydrolysates of Example 12.
- Fig. 13: shows the MS-spectra of rice hydrolysates of Example 13.
- Fig. 14: shows the evaluation of collagen protein level modulated by trans-epithelial transported digested and not digested peptides of Example 14.
- Fig. 15: shows the evaluation of collagen secretion modulated by trans-epithelial transported digested and not digested peptides of Example 15.
- Fig. 16: shows the evaluation of elastin protein level modulated by trans-epithelial transported digested and not digested peptides of Example 16.
- Fig. 17: shows the evaluation of elastin secretion modulated by trans-epithelial transported digested and not digested peptides of Example 17.
- Fig. 18: shows the results of the viability assay on BJ-5ta cells with peptides CR1 to CR3, CP1 to CP5 of Example 18.
- Fig. 19: shows the results of the ABTS assay with peptides CP1, CP2 and CR1 of Example 19.
- Fig. 20: shows the results of the FRAP assay with CP1, CP2, CP5 and CR1 of Example 20.
- Fig. 21: shows the evaluation of the protein levels modulation of collagen with specific peptides of Example 21 at a concentration of 250 µM.
- Fig. 22: shows the evaluation of the protein levels modulation of collagen with specific peptides of Example 21 at a concentration of 500 µM.
- Fig. 23: shows the evaluation of the protein levels modulation of elastin with specific peptides of Example 22 at a concentration of 250 µM.
- Fig. 24: shows the evaluation of the protein levels modulation of elastin with specific peptides of Example 22 at a concentration of 500 µM.
- Fig. 25: shows the evaluation of the ECM stability (collagen secretion) with specific peptides of Example 23.
- Fig. 26: shows the evaluation of the ECM stability (elastin secretion) with specific peptides of Example 23.

### DETAILED DESCRIPTION

The present invention will be better understood from the following description.

In this specification where reference is made to particular features of the invention it is to be understood that the disclosure of the invention in this specification includes all appropriate combinations of such particular features. The embodiments disclosed in this specification are exemplary and do not limit the invention. As used in this specification, the singular forms "a", "an", and "the" include plural reference unless the context clearly dictates otherwise. The terms "comprises" and "contains" are used in this specification to mean that, in addition to the features specifically identified, other features are optionally present. These terms are thus synonymous to "include". The term "at least" followed by a number is used herein to denote the start of a range beginning with that number. It is further noted that the following aspects and embodiments of the invention can be freely combined with each other. That is, the description is to be understood as also disclosing any combination of embodiments of the invention, except when a combination of embodiments does not make sense from a technical point of view.

In a first aspect, the present invention thus provides a composition comprising at least a first peptide of a first vegetable origin having skin physiology and/or morphology modifying and/or anti-aging properties. As used herein, the term "at least a first peptide of a first vegetable origin" means that the claimed composition can contain one or more peptides of the same vegetable origin, i.e. they can stem from the same plant (e.g. rice or pea). Further, "peptide" as used herein is a chain of 2 to 50 amino acids.

In an embodiment of the invention, the composition further contains at least a second peptide of a second vegetable origin having skin physiology and/or morphology modifying and/or anti-aging properties. As used herein, also the term "at least a second peptide of a second vegetable origin" means that the claimed composition can contain one or more peptides of the same vegetable origin, i.e. they can stem from the same plant (e.g. rice or pea).

Preferably, the at least first peptide and the at least second peptide are of different vegetable origin. This means that a composition of the claimed invention can comprise several peptides of one or two different vegetable origins. For example, a composition of the claimed invention can contain five peptides derived from pea and three peptides derived from rice.

The at least first peptide of a first vegetable origin is bioactive. In particular, it has skin physiology and/or morphology modifying and/or anti-aging properties. These properties include antioxidative properties as well as elastin and collagen protein level modulation and secretion.

In one embodiment, the at least first peptide of a first vegetable origin has antioxidative properties. These can be determined in commonly used *in vitro* methods, such as in ABTS (2,2-azino-bis-3-ethylbenzothiazoline-6-sulphonic acid) assay or a FRAP (ferric reducing antioxidant power) assay. In the ABTS assay 2,2-azino-bis-3-ethylbenzothiazoline-6-sulphonic acid is converted to a radical cation by addition of a persulfate. The radical cation absorbs light at 734 nm, in water. When antioxidants are added to a solution containing ABTS radical cation, the absorption decreases as the ABTS radical cation is terminated. The FRAP assay is a colorimetric assay that measures the ability of a sample to reduce the intense blue ferric tripyridyltriazine complex to its ferrous form, thereby changing its absorbance (Benzie and Strain, 1999).

In one embodiment, the ABTS radical scavenging activity of the at least first peptide is lower than 80% in an ABTS assay where a sample of the at least first peptide is compared to a control having an ABTS radical scavenging activity of 100%. For example, the ABTS radical scavenging activity of the at least first peptide is from 1 to 80%, preferably from 5 to 75%, more preferably from 7 to 60%, even more preferably from 10 to 50% in an ABTS assay where a sample of the at least first peptide is compared to a control having an ABTS radical scavenging activity of 100%. A method for determining the ABTS radical scavenging activity is defined in the experimental part.

In one embodiment, the ferric reducing antioxidant power of the at least first peptide is greater than 100% in a FRAP assay where a sample of the at least first peptide is compared to a control having a ferric reducing antioxidant power of 100%. For example, the ferric reducing antioxidant power of the at least first peptide is from 100 to 10000%, preferably from 200 to 8000%, more preferably from 250 to 7000%, even more preferably from 300 to 5000% in a FRAP assay where a sample of the at least first peptide is compared to a control having a ferric reducing antioxidant power of 100%. A method for determining the ferric reducing antioxidant power is defined in the experimental part.

In a further embodiment, the antioxidative activity of the at least first peptide is determined by measuring the modulation of ROS (Reactive Oxygen Species) levels, in particular the reduction of ROS levels, in BJ-5ta fibroblast cells in a respective assay in the presence of H₂O₂ as stimulant. In one embodiment, ROS production of the at least first peptide is lower than the one in a corresponding experiment using only H₂O₂ as stimulant. In one embodiment, ROS production is about 2000% using only H₂O₂ compared to a control (without stimulant and first peptide) having a ROS production of 100%. Thus, ROS production of the at least first peptide can be below 2000%. For example, ROS production of the at least first peptide may range from 100 to 1900%, preferably from 200 to 1700%, more preferably from 250 to 1500%, in a respective assay where a sample of the at least first peptide is compared to a control having a ROS production of 100%. A method for determining the ROS production is defined in the experimental part. In one embodiment, the reduction of ROS production caused by the at least first peptide compared to H₂O₂ is from 10 to 80%, as determined according to the method defined in the experimental part.

In a further embodiment, the antioxidative activity of the at least first peptide is determined by measuring the modulation of lipid peroxidation levels (an indicator for cellular membrane stability), in particular the reduction of lipid peroxidation levels, in BJ-5ta fibroblast cells in a respective assay in the presence of H₂O₂ as stimulant. In one embodiment, lipid peroxidation of the at least first peptide is lower than the one in a corresponding experiment using only H₂O₂ as stimulant. In one embodiment, lipid peroxidation is about 350 % using only H₂O₂ compared to a control (without stimulant and first peptide) having lipid peroxidation of 100%. Lipid peroxidation of the at least first peptide can be thus below 350%, for example, from 100 to 340%, preferably from 110 to 300%, more preferably from 120 to 250%, in a respective assay where a sample of the at least first peptide is compared to a control having a lipid peroxidation of 100%. A method for determining the lipid peroxidation is defined in the experimental part. In one embodiment, the reduction of lipid peroxidation caused by the at least first peptide compared to H₂O₂ is from 10 to 80%, as determined according to the method defined in the experimental part.

In a further embodiment, the at least first peptide is biocompatible. In other words, the at least first peptide is safe. Safety can be determined by measuring the cellular viability for example in a viability assay on BJ-5ta fibroblast cells. In an embodiment, the viability of BJ-5ta fibroblast cells treated with the at least first peptide in a respective assay is greater than 90% compared to the control. For example, the viability of BJ-5ta fibroblast cells treated with the at least first peptide in a respective assay is from 90% to 100%, preferably, 92% to 99.5%, more preferably 93 to 99% compared to the control. A method for determining the viability of BJ-5ta fibroblast cells is defined in the experimental part.

In one embodiment, the at least first peptide prevents or reduces extracellular matrix (ECM) degradation by modulating collagen and/or elastin levels, in particular by increasing collagen and/or elastin levels. In a further embodiment, the at least first peptide can prevent or reduce extracellular matrix (ECM) degradation by stimulating collagen and/or elastin secretion.

In one embodiment, the at least first peptide modulates, in particular increases, collagen protein levels. This modulation can be determined in a suitable assay. For example, collagen protein level/b-actin is higher than a corresponding experiment using only H₂O₂. In one embodiment, collagen protein level/b-actin is about 60 % using only H₂O₂. Collagen protein level/b-actin can thus be greater than 60% with the at least first peptide, for example, from 61 to 150%, preferably from 65 to 120%, more preferably from 70 to 100%, in a respective assay with the at least first peptide compared to a control having a collagen protein level/b-actin of 100%. A method for determining collagen protein level/b-actin is defined in the experimental part. In one embodiment, the collagen protein level/b-actin with the at least first peptide (in the presence of H₂O₂) is from 10 to 80% higher than the collagen protein level/b-actin with H₂O₂, as determined according to the method defined in the experimental part.

In one embodiment, the at least first peptide modulates elastin levels. This modulation can be determined in a respective assay. For example, elastin protein level/b-actin is higher than a corresponding experiment using only H₂O₂. In one embodiment, elastin protein level/b-actin is about 50 % using only H₂O₂. Elastin protein level/b-actin can thus be greater than 50% with the at least first peptide, for example, from 51 to 100%, preferably from 55 to 95%, more preferably from 60 to 92%, in a respective assay with the at least first peptide compared to a control having an elastin protein level/b-actin of 100%. A method for determining elastin protein level/b-actin is defined in the experimental part. In one embodiment, the increase of elastin protein level/b-actin caused by the at least first peptide compared to H₂O₂ is from 10 to 80%, as determined according to the method defined in the experimental part.

In one embodiment, the at least first peptide modulates protein levels of phospho(Ser473)-Akt or/and phospho(Thr172)-AMPK which are involved in extracellular matrix (ECM) degradation. This modulation can be determined in a suitable immunoblotting assay.

In one embodiment, phospho(Ser473)-Akt protein level/b-actin is lower than a corresponding experiment using only H₂O₂. In one embodiment, phospho(Ser473)-Akt protein level/b-actin is about 200 % using only H₂O₂. Phospho(Ser473)-Akt protein level/b-actin can thus be lower than 200% with the at least first peptide, for example, from 50 to 180%, preferably from 55 to 160%, more preferably from 60 to 130%, in a respective assay with the at least first peptide compared to a control having a phospho(Ser473)-Akt protein level/b-actin of 100%. A method for determining phospho(Ser473)-Akt protein level/b-actin is defined in the experimental part. In one embodiment, the reduction of phospho(Ser473)-Akt level/b-actin caused by the at least first peptide compared to H₂O₂ is from 10 to 80%, as determined according to the method defined in the experimental part.

In one embodiment, phospho(Thr172)-AMPK protein level/b-actin is lower than a corresponding experiment using only H₂O₂. In one embodiment, phospho(Thr172)-AMPK protein level/b-actin is about 130 % using only H₂O₂. Phospho(Thr172)-AMPK protein level/b-actin can thus be lower than 130% with the at least first peptide, for example, from 50 to 120%, preferably from 55 to 110%, more preferably from 60 to 100%, in a respective assay with the at least first peptide compared to a control having a phospho(Thr172)-AMPK level/b-actin of 100%. A method for determining phospho(Thr172)-AMPK protein level/b-actin is defined in the experimental part. In one embodiment, the reduction of phospho(Thr172)-AMPK level/b-actin caused by the at least first peptide compared to H₂O₂ is from 10 to 80%, as determined according to the method defined in the experimental part.

In one embodiment, the at least first peptide stimulates collagen and/or elastin secretion. This modulation can be determined in an ELISA assay. In one embodiment, collagen secretion is higher than a corresponding experiment using only H₂O₂. In one embodiment, collagen secretion is about 75 % using only H₂O₂, compared to a control having a collagen secretion of 100%. Collagen secretion can thus be greater than 75% with the at least first peptide, for example, from 80 to 200%, preferably from 85 to 170%, more preferably from 90 to 140%, in a respective assay with the at least first peptide compared to a control having a collagen secretion of 100%. A method for determining the collagen secretion is defined in the experimental part.

In one embodiment, elastin secretion is higher than a corresponding experiment using only H₂O₂. In one embodiment, elastin secretion is about 50 % using only H₂O₂, compared to a control having a collagen secretion of 100%. Elastin secretion can thus be greater than 50% with the at least first peptide, for example, from 55 to 100%, preferably from 60 to 90%, in a respective assay with the at least first peptide compared to a control having an elastin secretion of 100%. A method for determining the elastin secretion is defined in the experimental part.

In one embodiment, the at least first peptide inhibits the secretion of matrix metalloproteinases (MMPs) which are involved in ECM degradation. The matrix metalloproteinases constitute a multigene family of over 25 secreted and cell surface enzymes that process or degrade numerous pericellular substrates. Their targets include other proteinases, proteinase inhibitors, clotting factors, chemotactic molecules, latent growth factors, growth factor-binding proteins, cell surface receptors, cell-cell adhesion molecules, and virtually all structural extracellular matrix proteins. Thus, MMPs are able to regulate many biologic processes and are closely regulated themselves. This inhibition can be determined in a respective ELISA assay. In one embodiment, MMP-2 secretion is lower than a corresponding experiment using only H₂O₂. In one embodiment, MMP-2 secretion is about 120 % using only H₂O₂, compared to a control having a collagen secretion of 100%. MMP-2 secretion can thus be lower than 120% with the at least first peptide, for example, from 50 to 115%, preferably from 70 to 110%, more preferably from 80 to 100%, in a respective assay with the at least first peptide compared to a control having a MMP-9 secretion of 100%. A method for determining MMP-2 secretion is defined in the experimental part. In one embodiment, the reduction of MMP-2 secretion caused by the at least first peptide compared to H₂O₂ is from 10 to 80%, as determined according to the method defined in the experimental part.

In one embodiment, MMP-9 secretion is lower than a corresponding experiment using only H₂O₂. In one embodiment, MMP-9 secretion is about 140 % using only H₂O₂, compared to a control having a collagen secretion of 100%. MMP-9 secretion can thus be lower than 140% with the at least first peptide, for example, from 70 to 135%, preferably from 75 to 130%, more preferably from 80 to 110%, in a respective assay with the at least first peptide compared to a control having a MMP-9 secretion of 100%. A method for determining MMP-9 secretion is defined in the experimental part. In one embodiment, the reduction of MMP-9 secretion caused by the at least first peptide compared to H₂O₂ is from 10 to 80%, as determined according to the method defined in the experimental part.

In one embodiment, the at least first peptide has anti-inflammatory properties. In one embodiment, the at least first peptide modulates the levels of NF-kB. NF-kB is a transcription factor that plays a crucial role in various biological processes, including immune response, inflammation, cell growth, cell survival, and cell development. NF-κB is activated by various inflammatory stimuli such as growth factors and infectious microbes.

In one embodiment, NF-kB/b-actin is lower than a corresponding experiment using only H₂O₂. In one embodiment, NF-kB/b-actin is about 140 % using only H₂O₂, compared to a control having a NF-kB/b-actin value of 100%. NF-kB/b-actin can thus be lower than 140% with the at least first peptide, for example, from 100 to 135% in a respective assay with the at least first peptide compared to a control having a NF-kB/b-actin value of 100%. A method for determining NF-kB/b-actin is defined in the experimental part. In one embodiment, the reduction of NF-kB/b-actin caused by the at least first peptide compared to H₂O₂ is from 10 to 80%, as determined according to the method defined in the experimental part.

In a further embodiment, the at least first peptide inhibits the secretion of pro-inflammatory cytokines, such as Tnf-a, IL-6 and IL-1. Tumour Necrosis Factor a (Tnf-a), is an inflammatory cytokine produced by macrophages/monocytes during acute inflammation and is responsible for a diverse range of signaling events within cells, leading to necrosis or apoptosis. The protein is also important for resistance to infection and cancers. IL-6 is a cytokine featuring pleiotropic activity; it induces synthesis of acute phase proteins such as CRP, serum amyloid A, fibrinogen, and hepcidin in hepatocytes, whereas it inhibits production of albumin. IL-6 also plays an important role on acquired immune response by stimulation of antibody production and of effector T-cell development. Moreover, IL-6 can promote differentiation or proliferation of several nonimmune cells. IL-1 family is a group of 11 cytokines, which induces a complex network of proinflammatory cytokines and via expression of integrins on leukocytes and endothelial cells, regulates and initiates inflammatory responses. A method for determining Tnf-a, IL-6 and IL-1 secretion is defined in the experimental part. In this method, H₂O₂ stimulates the secretion of Tnf-a, IL-6 and IL-1, which is greater than 150% compared to a control having a secretion of Tnf-a, IL-6 and IL-1 of 100%. Thus, in one embodiment the secretion of Tnf-a, IL-6 and/or IL-1 in the presence of the at least first peptide is lower than with H₂O₂ alone. In one embodiment, the reduction of the secretion of Tnf-a, IL-6 and/or IL-1 caused by the at least first peptide compared to H₂O₂ is from 10 to 80%, as determined according to the method defined in the experimental part.

As explained above, the composition of the present invention can contain only one first peptide of a first vegetable origin. In that case, the one first peptide can have one or more of the properties described above, preferably more than one of the properties described above.

In one embodiment, the composition of the present invention contains more than one first peptide of a first vegetable origin having skin physiology and/or morphology modifying and/or anti-aging properties. In that case, each first peptide can independently have one or more of the properties described above.

In a further embodiment of the invention, the composition further contains at least a second peptide of a second vegetable origin having skin physiology and/or morphology modifying and/or anti-aging properties. As regards the properties of the at least second peptide of a second vegetable origin, the same applies as for the at least first peptide of a first vegetable origin defined above.

In one embodiment, the composition of the present invention contains more than one second peptide of a second vegetable origin having skin physiology and/or morphology modifying and/or anti-aging properties. In that case, each second peptide can independently have one or more of the properties described above.

As understood herewith, the term "vegetable origin" means that the peptides can be found in plants. It does, not, however, mean that the peptide(s) contained in the composition of the present invention must be obtained from plants. In other words, the peptide(s) contained in the composition of the present invention do not have to be obtained by a method involving a processing of the plants containing the peptide(s). The peptide(s) contained in the composition of the present invention can be produced synthetically. In addition, the at least "first peptide of a first vegetable origin" and the at least "second peptide of a second vegetable origin" means that the at least first and the at least second peptide have a different vegetable origin. Peptides originating from different varieties of subspecies of the same plant have the same vegetable origin, according to the present invention.

In an embodiment of the invention, the at least first peptide and the at least second peptide are a peptide originating from a cereal or a fabacea.

If the composition of the present invention contains at least a first peptide of a first vegetable origin and at least a second peptide of a second vegetable origin, it is preferred that at the least first peptide originates from a cereal and the at least second peptide originates from a fabacea, or vice versa.

In one embodiment, the cereal is selected from the group comprising rice, wheat, rye, oats, barley, millet, and maize. Preferably, the cereal is rice *(Oryza sativa).*

In one embodiment of the invention, the fabacea is selected from the group comprising soybean, bean, pea, chickpea, broad bean, alfalfa, peanut, carob, fenugreek, liquorice. Preferably, the fabacea is pea (*Pisum sativum*)*.*

If the composition of the present invention contains at least a first peptide of a first vegetable origin and at least a second peptide of a second vegetable origin, it is preferred that the at least first peptide originates from rice (*Oryza sativa*) and the at least second peptide originates from pea (*Pisum sativum*)*,* or vice versa.

In one embodiment of the present invention, the at least first peptide and/or the at least second peptide is a hydrolyzed peptide derived from, obtained from or contained in a hydrolysate. "Derived from a hydrolysate" means in the context of the present invention that a peptide is detected in a plant hydrolysate following a hydrolyzation process. This peptide is not isolated from the hydrolysate to be added to the composition of the present invention. On the contrary, the peptide is synthetically produced and then added to the composition of the present invention. "Obtained from a hydrolysate" means in the context of the present invention that a peptide is detected in a plant hydrolysate following a hydrolyzation process. This peptide is then isolated from the hydrolysate and is then added to the composition of the present invention. "Contained in a hydrolysate" means in the context of the present invention that a peptide is detected in a plant hydrolysate following a hydrolyzation process. This peptide is not isolated from the hydrolysate. Instead, the hydrolysate containing the peptide is added to the composition of the present invention. In this case, the hydrolysate can be processed for use in the composition of the present invention, e.g. by filtration (such as microfiltration, ultrafiltration, and/or nanofiltration). Preferably, in the composition of the present invention, the at least first peptide is a hydrolyzed peptide contained in a first hydrolysate and/or the at least second peptide is a hydrolyzed peptide contained in a second hydrolysate. More preferably, in the composition of the present invention, the at least first peptide is a hydrolyzed peptide contained in a first hydrolysate and the at least second peptide is a hydrolyzed peptide contained in a second hydrolysate.

In one embodiment, the hydrolysate has a peptide content of at least 50% (w/w), preferably 60 to 95 %(w/w).

In one embodiment, the hydrolysate has a molecular weight distribution in which at least 10% of the peptides have a molecular weight < 1000 Da and at least 30% of the peptides have a molecular weight < 5000 Da.

In a further embodiment, the hydrolysate has a molecular weight distribution in which at least 25% of the peptides have a molecular weight < 1000 Da and at least 50% of the peptides have a molecular weight < 5000 Da.

In yet a further embodiment, the hydrolysate has a molecular weight distribution in which at least 35% of the peptides have a molecular weight < 1000 Da and at least 60% of the peptides have a molecular weight < 5000 Da.

In one embodiment, the at least first peptide or the at least second peptide is a peptide derived from, obtained from or contained in a pea hydrolysate, wherein the at least first peptide or the at least second peptide is one or more of the peptides having SEQ-ID:1 to SEQ-ID:42. Preferably, the at least first peptide or the at least second peptide is one or more of the peptides having SEQ-ID: 15, SEQ-ID:20, SEQ-ID:22, SEQ-ID:23, SEQ-ID: 24, SEQ-ID:26, SEQ-ID:31, SEQ-ID:35, SEQ-ID:36, SEQ-ID:37, SEQ-ID:38, SEQ-ID:39, SEQ-ID:40, SEQ-ID:41, SEQ-ID:42. More preferably, the at least first peptide or the at least second peptide is one or more of the peptides having SEQ-ID:22, SEQ-ID:23, SEQ-ID:24, SEQ-ID:26, SEQ-ID:31.

In one embodiment, the at least first peptide or the at least second peptide is a peptide derived from, obtained from or contained in a rice hydrolysate, wherein the at least first peptide or the at least second peptide is one or more of the peptides having SEQ-ID:43 to SEQ-ID:62. Preferably, the at least first peptide or the at least second peptide is one or more of the peptides having SEQ:ID 43, SEQ-ID:45, SEQ-ID:48, SEQ-ID:55 to SEQ-ID:62. More preferably, the at least first peptide or the at least second peptide is one or more of the peptides having SEQ:ID 43, SEQ-ID:45, SEQ-ID:48.

In one embodiment of the present invention, the composition contains at least a first peptide being one or more of the peptides having SEQ-ID:1 to SEQ-ID:42 and at least a second peptide being one or more of the peptides having SEQ-ID:43 to SEQ-ID:62.

In one embodiment of the present invention, the composition contains at least a first peptide being one or more of the peptides having SEQ-ID:1 to SEQ-ID:42 and at least a second peptide being one or more of the peptides having SEQ-ID:43 to SEQ-ID:62.

In one embodiment of the present invention, the composition contains peptides having SEQ-ID:1 to SEQ-ID:42 and peptides having SEQ-ID:43 to SEQ-ID:62.

In one embodiment of the present invention, the at least first peptide comprises at least one of the three rice peptides with SEQ-:ID: 43, SEQ-ID:45 and SEQ-ID:48 and the at least second peptide comprises at least one of the five pea peptides having SEQ-ID:22, SEQ-ID:23, SEQ-ID:24, SEQ-ID:26, SEQ-ID:31.

In one embodiment of the invention, the composition contains peptides having SEQ-ID:22, SEQ-ID:23, SEQ-ID:24, SEQ-ID:26, SEQ-ID:31 and peptides having SEQ:ID 43, SEQ-ID:45, SEQ-ID:48.

In one embodiment, the composition is to be administered orally or topically, preferably orally. In case of oral administration, the composition can be in the form of a powder, granulate, solution, emulsion, suspension, tablet, chewable tablet, bar or capsule, drinking preparations, preferably a solution, emulsion or suspension. In case of topical administration, it is in the form of aqueous solutions, hydroalcoholic solutions, oil-in-water emulsions (O/W) or water-in-oil emulsions (W/O) or multiple emulsions (triple: W/O/W or O/W/O), or micro- or nano-emulsions.

In one embodiment, the composition of the present invention is a cosmetic or cosmeceutical composition, in particular for human or animal use.

In one embodiment, the cosmetic or cosmeceutical composition is an anti-aging composition which prevents, relieves, attenuates, alleviates, at least partially reverses, or treats the physical consequences of aging.

In one embodiment, the compositions according to the invention are useful to alleviate or at least partially reverse at least one sign of skin aging.

As intended herein a "sign of skin aging" relates to a skin defect which occurs as consequence of a degradation of skin constituents due to chronic factors, such as mechanical, oxidative and/or photo stresses. Skin aging can particularly be a consequence of chronological aging and/or photo-aging. "Chronological aging" relates to skin defects which occur as a consequence of age. "Photo-aging" relates to skin defects which occur as a consequence of skin exposition to light, and in particular to UV rays, more particularly UV-A or UV-B rays.

The at least one sign of skin aging may notably be at least one wrinkle or loss of skin elasticity. In particular the at least one sign of skin aging is at least one sign of facial skin aging.

The at least one sign of skin aging alleviated or at least partially reversed according to the invention is preferably at least one wrinkle, in particular at least one mimic wrinkle and/or mechanical fold, more particularly selected from the group consisting of forehead lines, frown lines, crow's feet wrinkles, bunny lines, purse-string wrinkles, nasolabial folds, tear troughs, marionette lines, mental crease and neck lines.

Where the cosmetic or cosmeceutical composition according to the invention is intended for topical administration to the skin, it may be in the form of any preparation conventionally used for topical application. In particular, it may advantageously be in the form of aqueous solutions, hydroalcoholic solutions, oil-in-water emulsions (O/W) or water-in-oil emulsions (W/O) or multiple emulsions (triple: W/O/W or O/W/O), or micro- or nano-emulsions.

When the cosmetic or cosmeceutical composition is in aqueous form, in particular in a dispersion, emulsion or aqueous solution, it may comprise an aqueous phase containing water.

In a preferred embodiment, the cosmetic or cosmeceutical composition is to be administered orally. Thus, the cosmetic or cosmeceutical composition can be in the form of a powder, granulate, solution, emulsion, suspension, tablet, chewable tablet, bar or capsule, drinking preparations, preferably a solution, emulsion or suspension. When the cosmetic composition is for oral administration, it can contain the same excipients as a nutritional or nutraceutical composition or a nutritional or food supplement, i.e. it can contain at least one nutritionally acceptable excipient and/or at least one supplementary food component as defined below.

In a further embodiment, the composition of the present invention is a nutritional composition or a dietary or food supplement or food for special medical purposes, in particular intended for human or animal consumption. Preferably, the composition is for use as a dietary or food supplement. In a further embodiment, the composition of the present invention is a nutraceutical composition, in particular intended for human or animal consumption. That is, the composition is for use as a nutraceutical.

In a preferred embodiment, the nutritional composition, the dietary or food supplement, the food for special medical purposes, or the neutraceutical composition is to be administered orally. Thus, nutritional composition, the dietary or food supplement or the neutraceutical composition can be in the form of a powder, granulate, solution, emulsion, suspension, tablet, chewable tablet, bar or capsule, drinking preparations, preferably a solution, emulsion or suspension.

Preferably, the composition as defined above further comprises at least one cosmetically or nutritionally acceptable excipient.

As intended herein, a cosmetically acceptable excipient is compatible with the skin, it is preferably of pleasant colour, smell and consistency and does not generate discomfort such as prickling, redness or other discomfort likely to discourage consumers from using it.

A cosmetically acceptable excipient according to the invention notably includes diluting agent, dispersing agent, gelling agent, solid emollient, gums, resins, solvents, fillers such as modified and polymerised starches, or metal stearate, preserving agents, essential oils, pearling agents, colouring agents, odour absorbers, pH regulators or neutralising agents, thickening agents, absorption-promoting agents and in particular ethanol and/or phospholipids, flavouring or perfuming agents, mineral pigments such as iron oxides, oil agents such as oils or fat of vegetable origin, fats of animal origin, synthetic oils, silicone oils (cyclomethicone) fluorine-containing oils, fatty alcohol esters (cetyl alcohol), waxes, modified clays, bentones, fatty acid metal salts, hydrophobized silica, polyethylenes, mica and/or other substances used in cosmetics.

The cosmetic or cosmeceutical composition according to the invention can be a care or make-up product for example in the form of a serum, lotion, cream, milk, water or oil gel, hydrogel, mask, stick, patch, oil, unguent, wax, foam, toner, care solution, balm, foundation or spray.

The cosmetic or cosmeceutical composition of the invention may further comprise other active molecules such as vitamins, sunscreens and filters, anti-age active substances, anti-oxidants, lightening agents, self-tanning ingredients, tanning accelerators, lifting ingredients, slimming agents, firming ingredients, hydrating ingredients, peeling ingredients, sebum-regulating agents, mattifying agents, etc. Preferably the cosmetic composition may comprise anti-oxidant agents, a lifting ingredient, firming ingredient and/or hydrating ingredient. In general, persons skilled in the art are able to select and adapt the quantities of additional active molecules so that the properties of the composition of the invention are not deteriorated through the addition thereof.

As intended herein a nutritionally acceptable excipient relates to a food component suitable for human or animal consumption.

In one embodiment, the nutritionally acceptable excipient is selected from the group consisting of anti-foaming agents, emulsifiers, firming agents, gelling agents, humectants, mineral salts, stabilizers, thickeners, and texturizing agent. Preferably, the anti-foaming agent is selected from the group consisting of polyethylene glycol and triethyl citrate. Preferably, the emulsifier is selected from lecithin, sorbitan monostearate and ammonium salts of phosphatidic acids. The firming agent is preferably selected from the group consisting of calcium chloride, calcium gluconate and calcium sulfate. The gelling agent is preferably selected from the group consisting of agar, calcium alginate and carrageenan. The humectant is preferably selected from the group consisting of glycerin, glycerol, lactitol and oxidized polyethylene; the mineral salt is cupric sulfate. Preferably, the stabilizer is selected from the group consisting of xanthan gum, guar gum and bleached starched. The thickener is preferably selected from the group consisting of tannins, sodium alginate and pectin. Preferably, the texturizing agent is selected from the group consisting of phosphates, sodium tripolyphosphate, sodium hexametaphosphate and sodium pyrophosphate.

In one embodiment, the composition further comprises at least one supplementary food component. Preferably, the supplementary food component is selected from the group consisting of vitamins, minerals, fibers, vegetable oil, fatty acids, amino acids, flavourings, colorants, antioxidants, sweeteners, acidity regulators, acidifiers, preservatives, sequestrants, seasonings, sugar, flour, prebiotics, salts, water, and antimicrobials. Preferably, the vitamin is selected from the group consisting of B vitamins including biotin, vitamin C and vitamin E. Fruit extracts and fruit flavors are used as natural flavourings. In particular, buckthorn, peach, passion fruit, passion fruit, blackberry, papaya and/or mango are used as fruits. The flavouring can also be oleoresin or aquaresins. The colorant is preferably selected from the group consisting of curcumin, brilliant blue, tartrazine and ferrous gluconate. Preferably, the antioxidant is selected from the group consisting of nitrates, nitrites, ascorbyl palmitate and calcium ascorbate. Preferably, the sweetener is selected from the group consisting of sorbitol, erythritol, xylitol, isomalt, alitame, aspartame, sodium cyclamate, acesulfame K, sucralose, saccharin, sodium saccharin, calcium saccharin, steviol glycosides, thaumatin and corn syrup. The acidity regulator is preferably selected from the group consisting of acetic acid, citric acid, fumaric acid, malic acid, lactic acid or tartaric acid. The acidifier is preferably lactic acid. The preservative is preferably selected from the group consisting of sodium nitrate, benzoic acid, sodium benzoate, tocopherols, ascorbic acid, niacin, riboflavin and thiamine. Preferably, the sequestrant is potassium gluconate. The seasoning is preferably selected from the group consisting of spices or oleoresins extracted from them, herbs, vegetables, essential oils, sodium nitrate, water, salt, sugars, and flavors. Preferably, the salt is selected from the group comprising sodium or potassium chloride, zinc in the form of a physiologically compatible zinc compound/zinc salt. Preferably, the antimicrobial is selected from the group consisting of lactic acid, citric acid, acetic acid, sodium diacetate, acidified sodium chloride or calcium sulfate, activated lactoferrin, sodium or potassium lactate, or bacteriocins such as nisin.

In one embodiment, the composition of the invention further contains at least one of:
- vitamin C,
- biotin,
- zinc in the form of a physiologically compatible zinc compound/zinc salt,
- vitamin E, and
- one or more further components selected from the group consisting of sugars, stabilizers, acidity regulators, preservatives and flavourings, and water.

Vitamin C, biotin (vitamin B7) and zinc are preferably used in a physiologically compatible form approved in accordance with EU Regulation 1925/2006, preferably as a combination of L-ascorbic acid and sodium L-ascorbate for vitamin C.

It is noted that the at least first peptide and the at least second peptide can be administered orally, as they are not digested or only partially digested in the stomach and in the intestine, thus keeping their bioactive properties.

In case of oral administration, the composition of the present invention, regardless of its use, is administered once or twice daily, preferably once daily. In case of an administration once a day, the composition of the present invention can be administered in the morning or in the evening, preferably in the morning.

The at least first peptide and the at least second peptide can be contained in the composition in a effective amount. The "effective" amount depends on various factors, such as age, the state of the subject, the severity of the disorder or condition and mode of administration. An effective amount means a nontoxic amount sufficient to produce the desired effect.

In any composition according to the invention, the at least first peptide and - if present - the at least second peptide to be present in an effective amount are generally in proportions of between 0.000001% and 15% relative to the total weight of the composition, more preferably between 0.0001 % and 5%, depending on the destination of the composition and the more or less pronounced desired effect. The at least first peptide and - if present - the at least second peptide may be present in the compositions according to the invention in varying relative proportions, in equivalent amounts, or on the contrary in different proportions.

As explained above, the at least first peptide and/or the at least second peptide is a hydrolyzed peptide derived from, obtained from or contained in a hydrolysate. Hence, in one embodiment of the composition of the present invention, the at least first peptide and/or the at least second peptide are obtained from a method comprising the following steps:
a) preparing a slurry of plant flour or proteins in an aqueous medium,
b) hydrolysing the plant flour or proteins by adding an enzyme to obtain a plant peptide hydrolysate,
c) optionally filtering the plant peptide hydrolysate through a first membrane (e.g. a microfiltration membrane) to give a first permeate and a first concentrate,
d) optionally filtering the first permeate through a second membrane (e.g. an ultrafiltration membrane) to give a second permeate and a second concentrate, and
e) optionally filtering the first or the second permeate through a third membrane (e.g. a nanofiltration membrane) to give a third permeate and a third concentrate.

In step a), plant proteins can be extracted from a plant source material by dry as well as humid approaches, as described in literature elsewhere. All methods of extraction or purification known to the person skilled in the art can be used for the preparation of the starting raw material. Otherwise, it is also possible to source commercial plant proteins.

In step b), the enzymes used in the hydrolysis step can be selected from fungal, microbial, and vegetal enzymes.

In one embodiment, the membrane filtration in optional step c) are replaced by other separation methods such as filtration through a plate and frame filter press, rotating drum filtration, centrifugation, sedimentation.

In one embodiment, the filtration in optional steps d) and e) are replaced by other separation methods, such as ion exchange chromatography and precipitation.

In one embodiment, the first permeate is a low molecular weight fraction comprising at least 11 wt.-% peptides within the 800 to 1900 Da range, wherein the second permeate is a low molecular weight fraction comprising at least 20 wt.-% peptides within the 800 to 1900 Da range and wherein the third permeate is a low molecular weight fraction comprising a higher weight percentage of peptides within the 800 to 1900 Da range than the first or second permeate.

A hydrolysate can be obtained by the following exemplary procedure: A plant protein slurry can be prepared by dispersing plant proteins in water (e.g. 10% w/v) to obtain a liquid suspension. The temperature of the slurry can be maintained at a temperature between 40 and 60°C. If necessary, the pH of the slurry can be corrected using corresponding agents. The plant protein slurry can then be subjected to hydrolysis by adding an enzyme for 3 hours of continuous stirring at the above temperature and pH. The slurry can then be optionally further hydrolysed by adding at least an additional enzyme under continuous stirring, and constant temperature and pH for an additional 2 hours. The enzyme mixture can be deactivated by lowering the pH with adequate acid solution, and/or by heating to 80°C - 100°C for 15 minutes to produce the raw plant protein hydrolysate which is then was cooled.

Another aspect of the invention relates to a composition comprising at least a first peptide originating from rice (*Oryza sativa*) and at least a second peptide originating from pea (*Pisum sativum*)*,* wherein the at least first peptide is obtained from a method comprising the following steps:
a') preparing a slurry of rice proteins in an aqueous medium,
b') hydrolysing the rice proteins by adding an enzyme to obtain a rice peptide hydrolysate,
c') filtering the rice peptide hydrolysate through a first membrane (e.g. a microfiltration membrane) to give a first permeate and a first concentrate,
d') filtering the first permeate through a second membrane (e.g. an ultrafiltration membrane) to give a second permeate and a second concentrate, and
e') optionally filtering the second permeate through a third membrane (e.g. a nanofiltration membrane) to give a third permeate and a third concentrate.

In one embodiment, the at least first peptide obtained by this method comprises at least one of peptides having the SEQ-ID:1 to SEQ-ID:42 and the at least a second peptide obtained by this method comprises at least one of peptides having the SEQ-ID:43 to SEQ-ID:62.

The at least second peptide originating from pea may be obtained analogous to the at least first peptide. For example, the at least second peptide may be obtained from a method comprising the following steps:
a") preparing a slurry of pea proteins in an aqueous medium,
b") hydrolysing the pea proteins by adding an enzyme to obtain a pea peptide hydrolysate,
c") filtering the pea peptide hydrolysate through a first membrane (e.g. a microfiltration membrane) to give a first permeate and a first concentrate,
d") filtering the first permeate through a second membrane (e.g. an ultrafiltration membrane) to give a second permeate and a second concentrate, and
e") optionally filtering the second permeate through a third membrane (e.g. a nanofiltration membrane) to give a third permeate and a third concentrate.

The enzyme used in step b") may be the same as the enzyme used in step b'). Preferably, however, the enzyme used in step b") is different from the enzyme used in step b'). In one embodiment, a mix of enzymes is added to the rice and pea proteins, respectively, in steps b") and b').

The composition comprising at least a first peptide originating from rice (*Oryza sativa*) and at least a second peptide originating from pea (*Pisum sativum*)*,* wherein the first and second peptides are obtained as described above, may be provided in the form of a clear and homogeneous aqueous solution with excellent stability. Preferably the composition can be stored in this form for several months without sedimentation. Furthermore, the composition is found to have a pleasant taste and is not perceived as bitter. This composition is suitable for oral administration, e.g. as a cosmetic, a cosmeceutical, a dietary or food supplements, food for special medical purposes, or as a nutraceutical.

In a second aspect, the present invention provides a peptide being one of the peptides having the SEQ-ID:1 to SEQ-ID:62 or a mixture of two or more of the peptides having the SEQ-ID:1 to SEQ-ID:62. These peptides are of vegetable origin. Peptides of SEQ-ID:1 to SEQ-ID:42 originate from pea (*Pisum sativum*)*,* whereas peptides of SEQ-ID:43 to SEQ-ID:62 originate from rice (*Oryza sativa*)*.*

The claimed peptides of vegetable origin were surprisingly found to have bioactive properties. In particular, the claimed peptides have skin physiology and/or morphology modifying and/or anti-aging properties, such as antioxidant activity, modulation of collagen and elastin protein levels as well as modulation of collagen and elastin secretion.

### EXAMPLES

The Examples set forth herein are meant to exemplify the various aspects of carrying out the invention and are not intended to limit the invention in any way. The following examples describe and demonstrate various aspects within the scope of the present invention. The examples are only given for illustrative purposes and should not be considered to be restrictive to this invention.

### ABTS Assay

The ABTS assay is based on the reduction of the 2,2-azino-bis-(3-ethylbenzothiazoline-6-sulfonic acid (ABTS) radical induced by antioxidants. The ABTS radical cation (ABTS+● was prepared by mixing a 7 mM ABTS solution (Sigma-Aldrich, Milan, Italy) with 2.45 mM potassium persulfate (1:1) and stored for 16 h at room temperature and in dark. To prepare the ABTS reagent, the ABTS+● was diluted in 5 mM phosphate buffer (pH 7.4) to obtain a stable absorbance of 0.700 (±0.02) at 730 nm. For the assay, 10 µL of samples were added to 140 µL of diluted the ABTS+● at the following final concentrations: 0.01 and 0.1 mg/mL for pea and rice peptides; 0.1, 1, 10 and 20 mg/mL for collagen peptides; 0.0001, 0.001 and 0.01 mg/mL for ascorbic acid; 1, 10, 25, 50, 100 µM for CR1, CP1 and CP2 peptides. The microplate was incubated for 30 min at 30 °C and the absorbance was read at 730 nm using a Synergy^{™} HT-multimode microplate reader (Biotek Instruments, Winooski, VT, USA). The TEAC values were calculated using a Trolox (Sigma-Aldrich, Milan, Italy) calibration curve (60-320 µM).

### FRAP Assay

The FRAP assay evaluates the ability of a sample to reduce ferric ion (Fe3⁺) into ferrous ion (Fe2⁺). Thus, 10 µL of samples were mixed with 140 µL of FRAP reagent until reaching the following concentrations: 0.01, 0.1 and 1 mg/mL for pea and rice peptides; 1, 10 and 20 mg/mL for collagen peptides; 0.00001, 0.0001, 0.001 and 0.1 mg/mL for ascorbic acid; 10, 100 and 200 µM for CR1 and CP5 peptides; 25, 50, 100 and 200 µM for CP1 and CP2 peptides. The FRAP reagent was prepared by mixing 1.3 mL of a 10 mM TPTZ (Sigma-Aldrich, Milan, Italy) solution in 40 mM HCl, 1.3 mL of 20 mM FeCl₃ × 6H₂O and 13 mL of 0.3 M acetate buffer (pH 3.6). The microplate was incubated for 30 min at 37 °C and the absorbance was read at 595 nm. The results were calculated by a Trolox (Sigma-Aldrich, Milan, Italy) standard curve obtained using different concentrations (3-400 µM)). Absorbances were recorded on a Synergy^{™} HT-multimode microplate reader.

### Cell culture conditions

Fibroblast cells (BJ-5ta, CRL-4001) were cultured in complete medium, a 4:1 mixture of Dulbecco's medium and Medium 199 with supplements as 4 parts of Dulbecco's Modified Eagle's Medium containing 4 mM L-glutamine, 4.5 g/L glucose and 1.5 g/L sodium bicarbonate, 1 part of Medium 199 supplemented with 0.01 mg/mL hygromycin B 10% fetal bovine serum with incubation at 37 °C under 5% CO₂ atmosphere.

### Caco-2 Cell Culture and Differentiation

Caco-2 cells, obtained from INSERM (Paris, France) were routinely sub-cultured at 50% confluence and maintained at 37°C in a 5% CO₂ atmosphere in DMEM containing 25 mM of glucose, 3.7 g/L of NaHCOs, 4 mM of stable L-glutamine, 1% non-essential amino acids, 100 U/L of penicillin and 100 µg/L of streptomycin (complete medium), supplemented with 10% heat-inactivated FBS. To facilitate the establishment of a confluent cell monolayer, cells were seeded on a Transwell at a density of 3.5 × 10⁵ cells/cm² in complete medium supplemented with 10% FBS in both the AP and BL compartments for a period of 2 days. Cells were seeded, and on day three, they were moved to an FBS-free medium in the AP compartment. They were then allowed to differentiate for 18-21 days, with three weekly medium changes in between. Transepithelial electrical resistance (TEER) of differentiated Caco-2 cells was measured at 37 °C using the Millicell voltmeter device (Millipore Co., Billerica, MA, USA) both before and after the transport experiments for monitoring the integrity of the cell monolayers.

### Trans-Epithelial Transport Experiments

TEER measurement was used to verify the integrity and differentiation of the cell monolayer prior to transport experiments. Pea and rice peptides trans-epithelial transit was assessed in differentiated Caco-2 cells in transport buffer solution (137 mM NaCl, 5.36 mM KCl, 1.26 mM CaCl2, and 1.1 mM MgCl2, 5.5 mM glucose). The apical (AP) solutions were kept at pH 6.0 (buffered with 10 mM morpholinoethane sulfonic acid) and the basolateral (BL) solutions were kept at pH 7.4 (buffered with 10 mM N-2-hydroxyethylpiperazine-N-4-butanesulfonic acid) in order to replicate the pH conditions found in vivo in the small intestinal mucosa. Before the transport assay, cells were equilibrated in HBSS for 15 minutes at 37°C. In the AP compartment with the AP transport solution (500 µL) and the BL compartment with the BL transport solution (700 µL), rice and pea (5 mg/mL) were added. All BL and AP solutions were collected after two hours of absorption experiment carried out at 37°C, and they were kept at - 80°C before analysis. Transport experiments were performed in duplicate.

### Cell Viability Assay - 3-(4,5-Dimethylthiazol-2-yl)-2,5-Diphenyltetrazolium Bromide (MTT) Assay

A total of 10⁴ fibroblasts/well were seeded in 96-well plates and treated with pea, rice and collagen peptides (from 1 to 20 mg/mL) and ascorbic acid (from 0.1 to 10 mg/mL), CR1, CR2, CP1, CP2, CP3, CP4 and CP5 peptides (from 25 to 500 µM) or H₂O₂ (from 0.1 to 1 mg/mL) or vehicle (H₂O) in complete growth media for 48 h at 37 °C under 5% CO₂ atmosphere. Subsequently, the treatment solvent was aspirated and 100 µL/well of 3-(4,5-dimethylthiazol-2-yl)-2,5-diphenyltetrazolium bromide (MTT) filtered solution added. After 2 h of incubation at 37 °C under 5% CO₂ atmosphere, 0.5 mg/mL solution was aspirated and 100 µL/well of the lysis buffer (8 mM HCl + 0.5% NP-40 in DMSO) added. After 10 min of slow shaking, the absorbance at 575 nm was read on the Synergy H1 fluorescence plate reader (Biotek, Bad Friedrichshall, Germany).

### Fluorometric Intracellular ROS Assay

For cells preparation, 10⁴ fibroblasts/well were seeded on a black 96-well plate overnight in growth medium. The day after, the medium was removed and replaced with 50 µL/well of complete DMEM and 50 µL/well of the Master Reaction Mix and the cells were incubated at 5% CO₂, 37 °C for 1 h in the dark. Then, cells were treated with 10 µL of 11x pea, rice and collagen peptides (to reach the final concentrations of 1, 5 and 7.5 mg/mL for pea peptides, 1 and 5 mg/mL for rice peptides ) and incubated at 37 °C for 24 h in the dark. To induce ROS, cells were treated with 10 µL of H₂O₂ at a final concentration of 200 µM for 1 h a 37°C in the dark and fluorescence signals (ex./ em. 490/525 nm) were recorded using a Synergy H1 microplate reader.

### Lipid peroxidation (MDA) assay

BJ-5ta cells (2.5 × 10⁵ cells/well) were seeded in a 24 well plate and, the following day, they were treated with pea peptides at final concentrations of 5 and 7.5 mg/mL, respectively, rice peptides at final concentration of 5.0 mg/mL and collagen peptides at final concentrations of 5 and 10 mg/mL, respectively, for 24 h at 37 °C under 5% CO₂ atmosphere. After incubation, cells were stimulated with H₂O₂ 200 µM, or vehicle, overnight, then collected and homogenized in 150 µL ice-cold MDA lysis buffer containing 1.5 µL of BHT (100X). Samples were centrifuged at 13,000 g for 10 min to remove insoluble material. To form the MDA-TBA adduct, 300 µL of the TBA solution were added into each vial containing samples and incubated at 95 °C for 60 min, then cooled to RT for 10 min in an ice bath. For analysis, 100 µL of each reaction mixture were pipetted into a 96 well plate and the absorbance was measured at 532 nm using the Synergy H1 fluorescent plate reader from Biotek.

### Western Blot Analysis

A total of 2.2 × 10⁵ BJ-5ta cells/well were seeded on 24-well plates and incubated at 37 °C under a 5% CO₂ atmosphere. The following day, cells were treated with pea, rice and collagen peptides (5 mg/mL), CR1, CR2, CP1, CP4 and CP5 (250 and 500 µM), CP2 (100, 250 and 500 µM) or vehicle (H₂O) in a complete growth medium for 24 h. The day after, cells were stimulated with H₂O₂ (200 µM) or vehicle (H₂O) for 1 h. After each treatment, cells were scraped in 30 µL ice-cold lysis buffer [RIPA buffer + inhibitor cocktail + 1:100 PMSF + 1:100 Na-orthovanadate] and transferred in an ice-cold microcentrifuge tube. After centrifugation at 13.300g for 15 min at 4 °C, the supernatant was recovered and transferred into a new ice-cold tube. Total proteins were quantified by Bradford method and 50 µg of total proteins loaded on a precast 7.5% Sodium Dodecyl Sulfate - Polyacrylamide (SDS-PAGE) gel at 130 V for 45 min. Subsequently, the gel was pre-equilibrated with 0.04% SDS in H₂O for 15 min at RT and transferred to a nitrocellulose membrane (Mini nitrocellulose Transfer Packs,) using a trans-Blot Turbo at 1.3 A, 25 V for 7 min. Target proteins, on milk or BSA blocked membrane, were detected by primary antibodies as follows: anti-collagen, anti-elastin, anti-phospho(Thr172)-AMPK, anti-phospho(Ser473)-Akt, anti-NF-κB and anti-β-actin. Secondary antibodies conjugated with HRP and a chemiluminescent reagent were used to visualize target proteins and their signal was quantified using the Image Lab Software (Biorad). The internal control β-actin was used to normalize loading variations.

### ELISA assay for pro-inflammatory and anti-inflammatory secreted cytokines, MMP-2, MMP-9, collagen and elastin quantification

Cytokines, metalloproteases, collagen and elastin quantification was performed using human Authentikine^{®} ELISA kits (Proteintech, Rosemont, USA) and Cusabio ELISA kits (Houston, TX, USA) according to the manufacturer's instruction and. Briefly, the supernatants collected from treated and H₂0-stimulated bj-5ta cells were centrifuged at 13,300 × g for 10 minutes at 4°C, then the pellet and insoluble material were discarded. For the experiments, 100 µL of samples were added to each well and the microplate was incubated for 2 hours at 37°C. After the incubation, the solutions were discarded and each well washed 4 times with 300 µL of Wash Buffer solution, then 100 µL of Detection antibody solution were added to each well and the microplate was incubated for 1 hours at 37°C. After incubation, the solutions were discarded and each well washed 4 times with 300 µL of Wash Buffer solution; than 100 µL of Streptavidin-HRP solution were added and the plate was incubated for 40 min at 37°C. After 4 washes, the TMB substrate (100 µL/well) was added and reactions were stopped after 20 min with 50 µL of Stop Solution and the absorbance at 450 and 540 nm was measured using the Synergy H1 plate reader (BioTek Instruments).

### Simulated gastrointestinal digestion with pepsin and trypsin enzymes

The enzymatic hydrolysis of pea and rice peptides was performed using pepsin and trypsin to simulate gastrointestinal digestion. For pepsin and trypsin digestion, the pH of the solution was adjusted to pH 2 and 8, respectively, by adding 1 M HCl or 1 M NaOH. The pepsin and trypsin solutions were added to the solution at a 1:100 (w/w) E/S ratio. After 60 min of peptic digestion at 37°C, pepsin was inactivated by adjusting the pH to 8; whereas the tryptic digestion (15 minutes) was blocked by heating the solution at 95 °C for 5 min (Lo et al., 2005). Collagen, pea and rice hydrolysates were passed through ultrafiltration membranes for the complete enzymes removal, using a Millipore UF system (Millipore, Bedford, MA, USA). Recovered peptides were lyophilized and stored at -80 °C for high-resolution LC-MS/MS analysis.

### Caco-2 and BJ-5ta cells co-culture experiment

For co-culture experiments, Caco-2 cells on filter inserts were transferred in multiwell culture plates containing confluent BJ-5ta cells. Prior to treatment with collagen, pea and rice digested and not digested peptides, differentiated Caco-2 cells were washed twice with 500 µL PBS with 1 mM Ca2+ and 1 mM Mg2+. The samples (5 mg/mL) were added in the complete medium (500 µL) of the AP compartment, whereas the BL compartment contained complete medium supplemented with 10% FBS (700 µL). After 2 h incubation of cells in co-culture, the AP solution and Caco-2 cells were discarded, while the plate containing BL media and BJ-5ta cells was incubated for 24h at 37°C in a 5% CO₂ atmosphere. Then, H₂O₂ 200 µM were added overnight and, at the end of the stimulation, cells media and cell lysates were collected for ELISA and western blot experiments, respectively.

### High-resolution LC-MS/MS Analysis and data elaboration

Each hydrolysate and peptide samples collected in AP and BL chamber, respectively, were desalted using zip-tip (C18) microcolumns, then dried and dissolved with 50 µL of a solution of 99% water and 1% ACN containing 0.1% formic acid. All samples have been analyzed using: Dionex Ultimate 3000 nano-LC system (Sunnyvale CA, USA) connected to Orbitrap Fusion^{™} Tribrid^{™} Mass Spectrometer (Thermo Scientific, Bremen, Germany) equipped with nanoelectrospray ion source.

### Statistical Analysis

All results were expressed as the mean ± standard deviation (s.d.), where p-values < 0.05 were considered to be significant. Statistical analyses were performed by Two-Way ANOVA followed by Šidák correction (GraphPad Prism 9, GraphPad Software, La Jolla, CA, USA).

### Example 1 - Evaluation of antioxidant activity of pea and rice peptides by ABTS

In this example, the antioxidant activity of pea and rice peptides was tested *in vitro* in an ABTS Assay as described above.

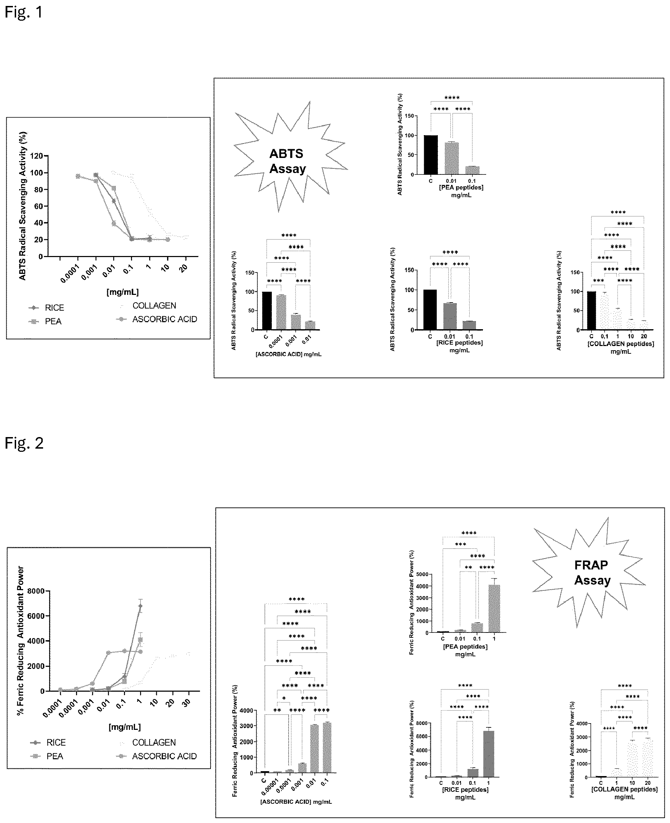

The results are shown in Fig. 1. It can be seen that both pea and rice peptides have antioxidant properties, as their ABTS radical scavenging activity is lower than the one of the control C. It is further noted that the ABTS radical scavenging activity decreases with increasing concentration of the peptides. The trend of decreasing radical scavenging activity with increasing concentration is also known from ascorbic acid and collagen protein (albeit this trend is observed at different concentrations, i.e. higher concentrations for collagen protein and lower concentrations for ascorbic acid when compared to pea and rice peptides).

### Example 2 - Evaluation of antioxidant activity of pea and rice peptides by FRAP

In this example, the antioxidant activity of pea and rice peptides was tested *in vitro* in a FRAP Assay as described above.

The results are shown in Fig. 2. It can be seen that both pea and rice peptides have antioxidant properties, as their ferric reducing antioxidant power is higher than the control C. It is further noted that the ferric reducing antioxidant power increases with increasing concentration of the peptides. An increase of the ferric reducing antioxidant power is also observed for ascorbic acid and collagen protein (again at different concentrations).

### Example 3 - Viability assay on BJ-5ta cells using pea and rice peptides

In this example, the effect of pea and rice peptides was tested on BJ-5ta cells in a modified MTT assay as described above. A cellular viability assay on skin fibroblasts exposed to increasing concentration of H₂O₂, through which damage/aging was induced, was also carried out.

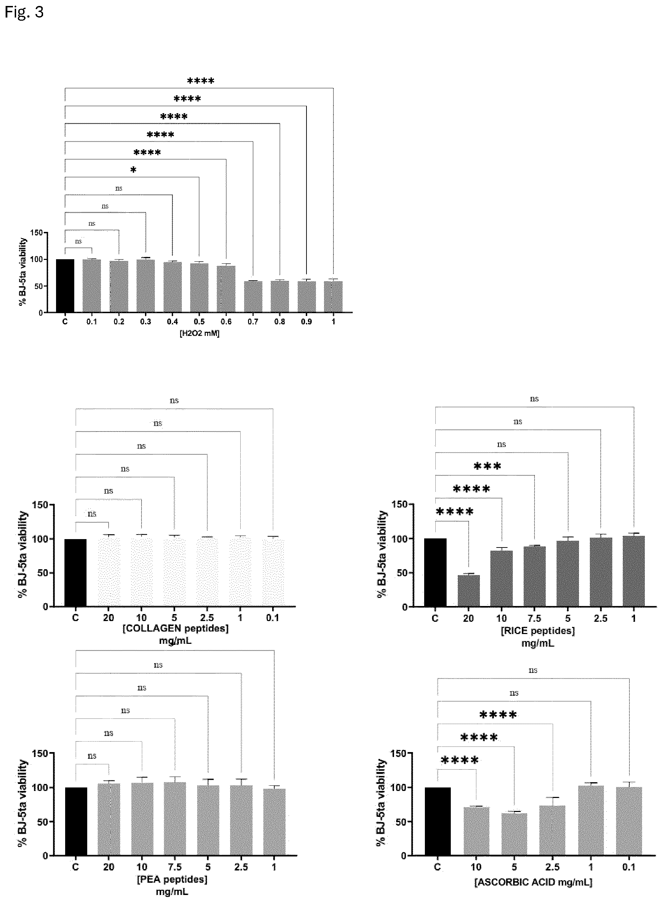

The results are shown in Fig. 3. Results show that there are no cytotoxic effects up to a concentration of H₂O₂ 0.4 mM. Results further show that pea and collagen peptides are safe at all the doses tested, while rice peptides do not have effects on cellular viability up to a concentration of 5 mg/mL. Ascorbic acid has effects on cellular viability in concentrations of more than 1 mg/mL.

### Example 4 - Modulation of ROS levels with pea and rice peptides

In this example, the antioxidant activity of pea and rice peptides was tested *in vitro* in a Fluorometric Intracellular ROS Assay as described above.

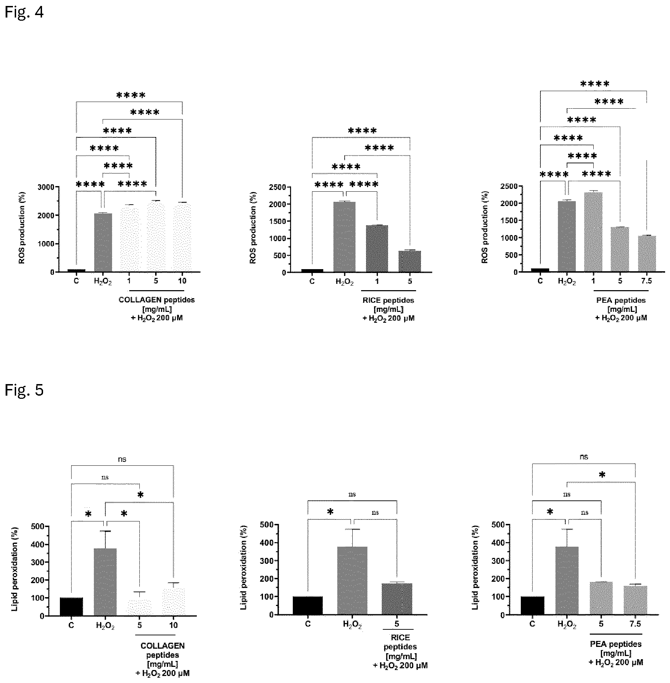

The results are shown in Fig. 4. Pretreatments (24 h) with pea and rice peptides significantly protected fibroblasts from the intracellular ROS increase, induced by H₂O₂ stimulation. However, a ROS reduction from collagen peptides was not observed.

### Example 5 - Modulation of lipid peroxidation with pea and rice peptides

In this example, the antioxidant activity of pea and rice peptides was tested *in vitro* in a MDAAssay as described above.

The results are shown in Fig. 5. Pretreatments (24 h) with pea, rice and collagen peptides significantly protected fibroblasts from lipid peroxidation, induced by H₂O₂ stimulation.

### Example 6 - Evaluation of the protein levels modulation of collagen and elastin with pea and rice peptides

In this example, the modulation of collagen and elastin protein levels with pea and rice peptides was tested *in vitro* by a Western Blot analysis as described above.

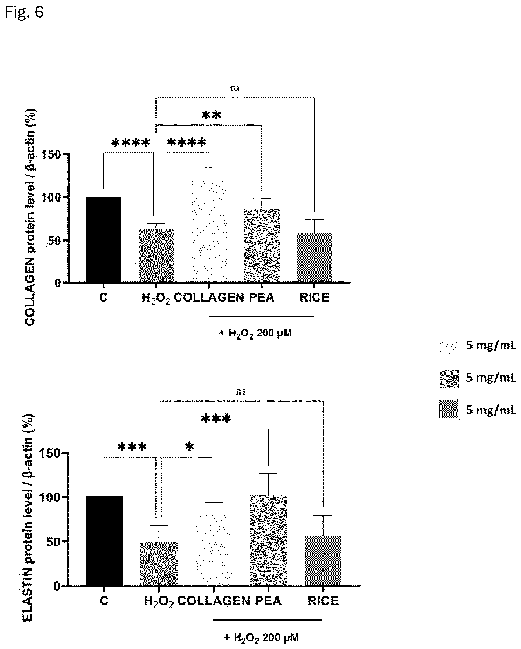

The results are shown in Fig. 6.

### Example 7 - Evaluation of the phospho(Ser473)-Akt and phospho(Thr172)-AMPK levels modulation with pea and rice peptides

In this example, the modulation of phospho(Ser473)-Akt and phospho(Thr172)-AMPK levels with pea and rice peptides was tested *in vitro* by a Western Blot analysis as described above.

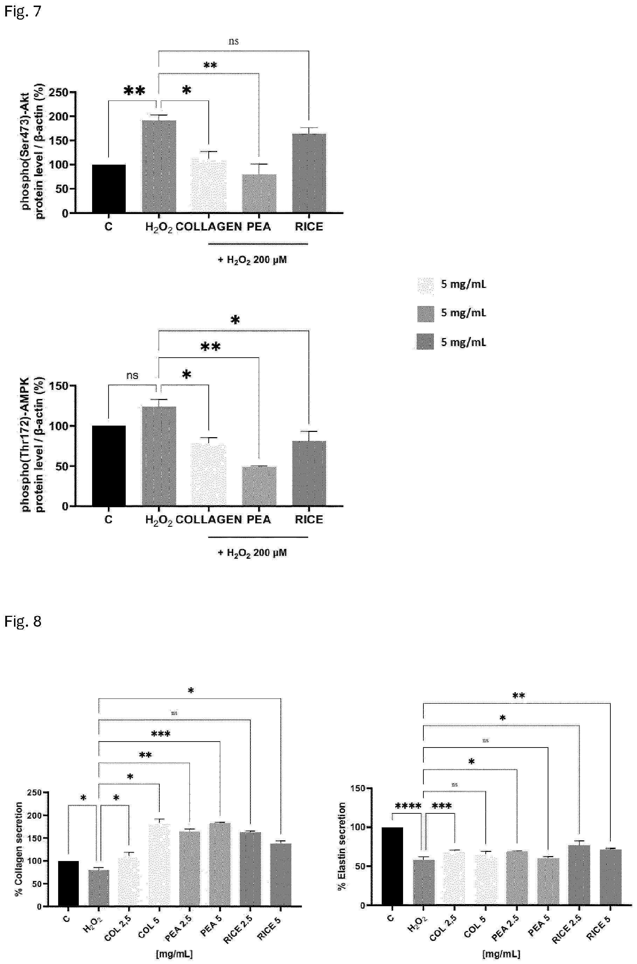

The results are shown in Fig. 7.

### Example 8 - Evaluation of the collagen and elastin secretion with pea and rice peptides

In this example, the secretion of collagen and elastin with pea and rice peptides was tested *in vitro* by an ELISA assay as described above. Thereby, ECM stability is evaluated.

The results are shown in Fig. 8. Pea and rice peptides significantly increase both the collagen and elastin secretion.

### Example 9 - Evaluation of the MMP-2 and MMP-9 secretion with pea and rice peptides

In this example, the secretion of MMP-2 and MMP-9 with pea and rice peptides was tested *in vitro* by an ELISA assay as described above. Thereby, ECM degradation is evaluated.

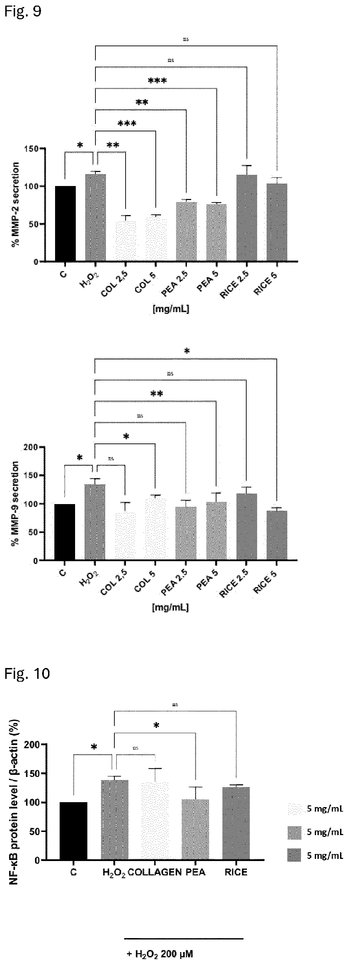

The results are shown in Fig. 9. Pea peptides significantly reduce both MMP-2 and MMP-9 secretion; rice peptides decrease MMP-9 secretion.

### Example 10 - Evaluation of the protein levels modulation of NF-κB with pea and rice peptides

In this example, the modulation of NF-κB levels with pea and rice peptides was tested *in vitro* by a Western Blot analysis as described above. Thereby, the anti-inflammatory activity is evaluated.

The results are shown in Fig. 10.

### Example 11 - Evaluation of the secretion of IL-10, IL-1, IL-6, TNF-α, IFN-ywith pea and rice peptides

In this example, the secretion of IL-10, IL-1, IL-6, TNF-α, IFN-γ with pea and rice peptides was tested *in vitro* by an ELISA assay as described above. Thereby, the anti-inflammatory activity is evaluated.

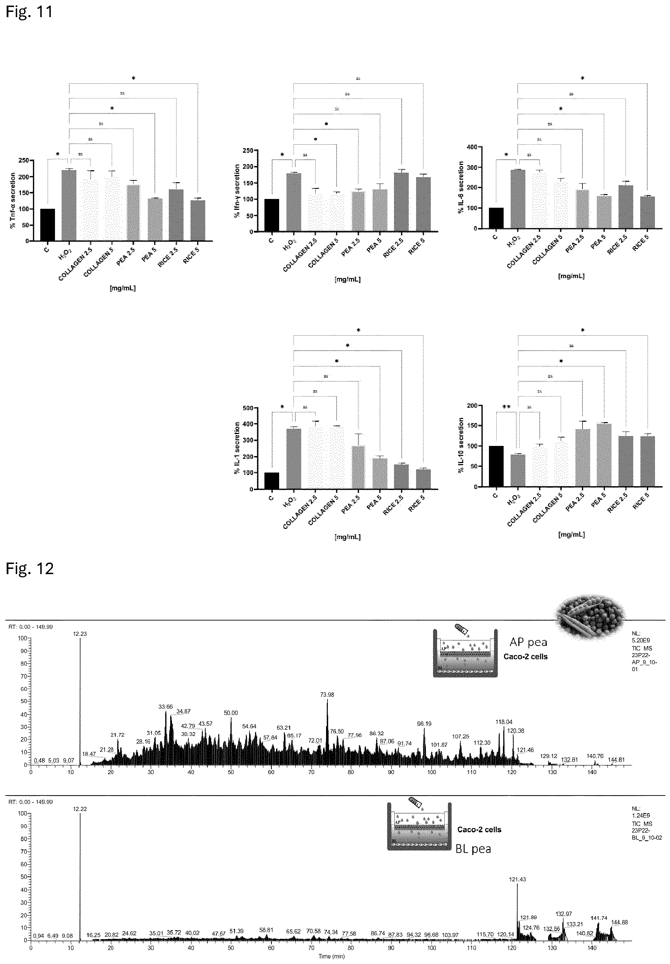

The results are shown in Fig. 11. Pea and rice peptides significantly decreased the Tnf-α, IL-6 and IL-11 and increased the IL-10 cytokines levels induced by H₂O₂ stimulation.

### Example 12 - Analysis of pea peptides

In this Example, pea peptides were analyzed by MS in a Caco-2 and BJ-5ta cells co-culture experiment as defined above (however without stimulation with H₂O₂). The samples were taken in the AP (apical) and BL (basolateral) chambers, respectively for the digested and non-digested pea hydrolysates.

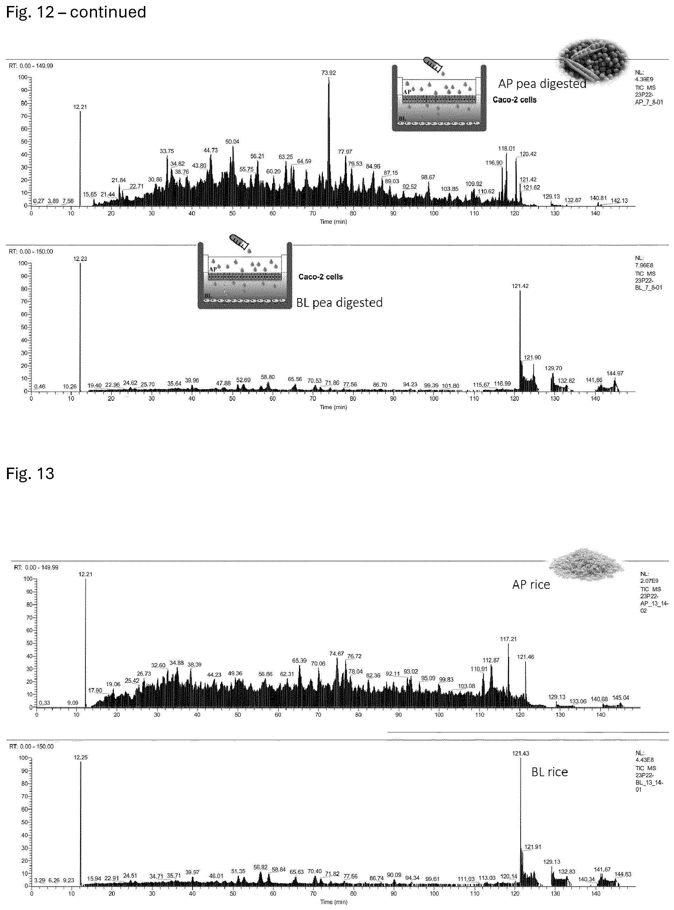

The MS spectra are shown in Fig. 12.

Peptides with SEQ-ID:1 to SEQ-ID:42 were identified.

When comparing AP pea digested vs. BL pea digested samples, 7 common sequences were found: SEQ-ID:2, SEQ-ID:23, SEQ-ID:24, SEQ-ID:26, SEQ-ID:15, SEQ-ID:12, SEQ-ID:35.

Peptides having SEQ-ID:2, SEQ-ID:23, SEQ-ID:24, SEQ-ID:26, SEQ-ID:12 were detected also in the AP pea and BL pea samples (i.e. in the non-digested samples).

### Example 13 - Analysis of rice peptides

In this Example, rice peptides were analyzed by MS in a Caco-2 and BJ-5ta cells co-culture experiment as defined above (however without stimulation with H₂O₂). The samples were taken in the AP and BL chambers, respectively for the digested and non-digested rice hydrolysates.

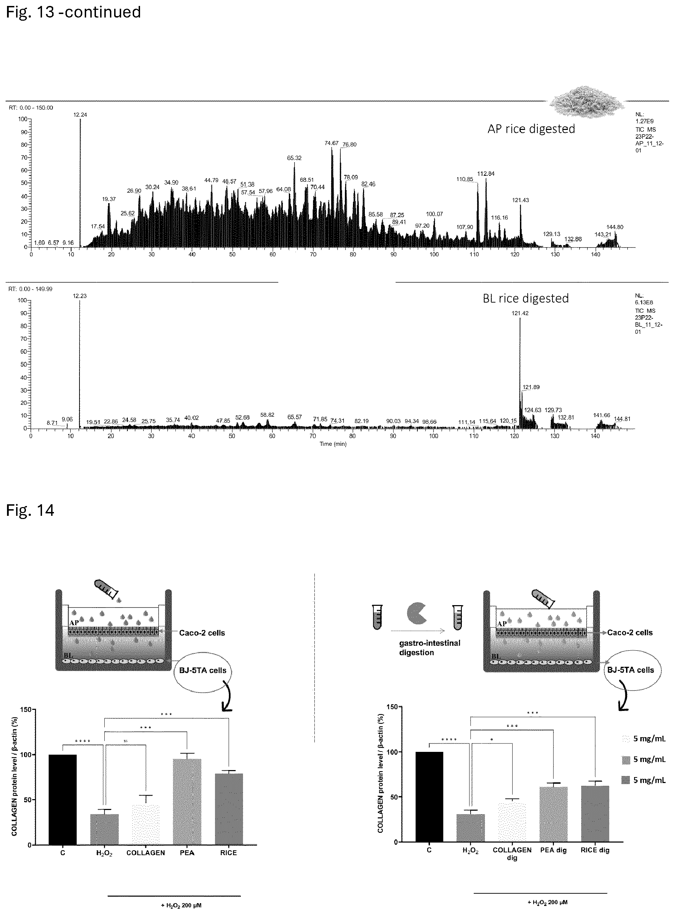

The MS spectra are shown in Fig. 13.

Peptides with SEQ-ID:43 to SEQ-ID:62 were identified.

When comparing BL rice digested vs. BL rice not digested samples, 3 common sequences were found: SEQ-ID:43, SEQ-ID:45 and SEQ-ID:48.

### Example 14 - Evaluation of collagen protein level modulated by trans-epithelial transported digested and not digested peptides

In this Example, collagen protein level modulated by trans-epithelial transported digested and not digested peptides were evaluated in a Caco-2 and BJ-5ta cells co-culture experiment as defined above.

The results are shown in Fig. 14. The capacity of pea and rice peptides (not digested and after their gastro-intestinal digestion) to modulate collagen production is confirmed.

### Example 15 - Evaluation of collagen secretion modulated by trans-epithelial transported digested and not digested peptides

In this Example, collagen secretion modulated by trans-epithelial transported digested and not digested peptides were evaluated in a Caco-2 and BJ-5ta cells co-culture experiment as defined above. Samples were taken from the BL chamber.

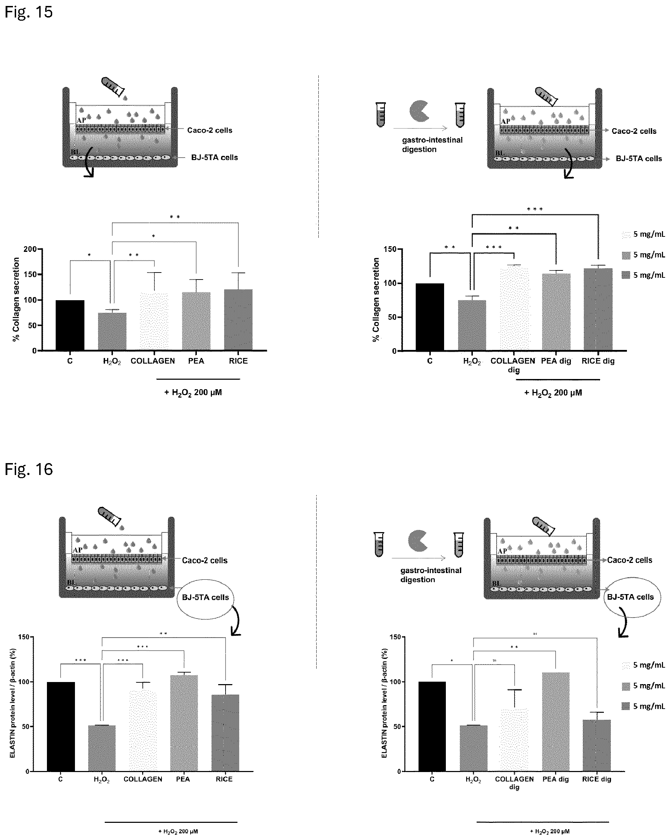

The results are shown in Fig. 15. The capacity of pea and rice peptides (not digested and after their gastro-intestinal digestion) to modulate collagen secretion is confirmed.

### Example 16 - Evaluation of elastin protein level modulated by trans-epithelial transported digested and not digested peptides

In this Example, elastin protein level modulated by trans-epithelial transported digested and not digested peptides were evaluated in a Caco-2 and BJ-5ta cells co-culture experiment as defined above.

The results are shown in Fig. 16. The capacity of pea and rice peptides (not digested and after their gastro-intestinal digestion) to modulate elastin production is confirmed.

### Example 17 - Evaluation of elastin secretion modulated by trans-epithelial transported digested and not digested peptides

In this Example, elastin secretion modulated by trans-epithelial transported digested and not digested peptides were evaluated in a Caco-2 and BJ-5ta cells co-culture experiment as defined above. Samples were taken from the BL chamber.

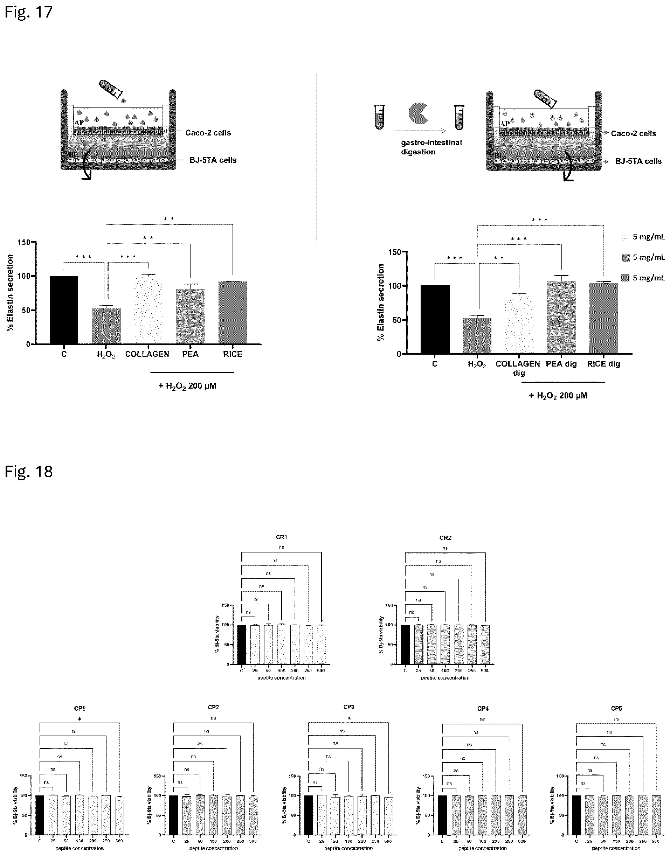

The results are shown in Fig. 17. The capacity of pea and rice peptides (not digested and after their gastro-intestinal digestion) to modulate elastin secretion is confirmed.

### Example 18 - Cell viability experiments with specific pea and rice peptides

Three rice peptides CR1-CR3 and five pea peptides having the sequences as defined below in Table 1 were synthesized.

**Table 1**

| **Name** | **Sequence** | **SEQ-ID** | **Mw g/mol** |
|---|---|---|---|
| CR1 | AEDVGEEVER | SEQ-ID:43 | 1132 |
| CR2 | AGFAGDDAPR | SEQ-ID:45 | 976 |
| CR3 | DWLLGATGTGK | SEQ-ID:48 | 1130 |
| CP1 | DTSETWAIR | SEQ-ID:22 | 1078 |
| CP2 | DWPEWVR | SEQ-ID:23 | 999 |
| CP3 | ENIADAAR | SEQ-ID:24 | 859 |
| CP4 | GSRQEEEEDEDEERQPR | SEQ-ID:26 | 2117 |
| CP5 | SSNNQLDQMPR | SEQ-ID:31 | 1289 |

With these peptides, cell viability experiments on BJ-5ta cells in a modified MTT assay were carried out as described above.

The results are shown in Fig. 18. Up to a concentration of 500 µM, no effects on cell viability were observed.

### Example 19 - Evaluation of antioxidant activity of CP1, CP2 and CR1 by ABTS

In this example, the antioxidant activity of peptides CP1, CP2 and CR1 (see Table 1 above) was tested *in vitro* in an ABTS Assay as described above.

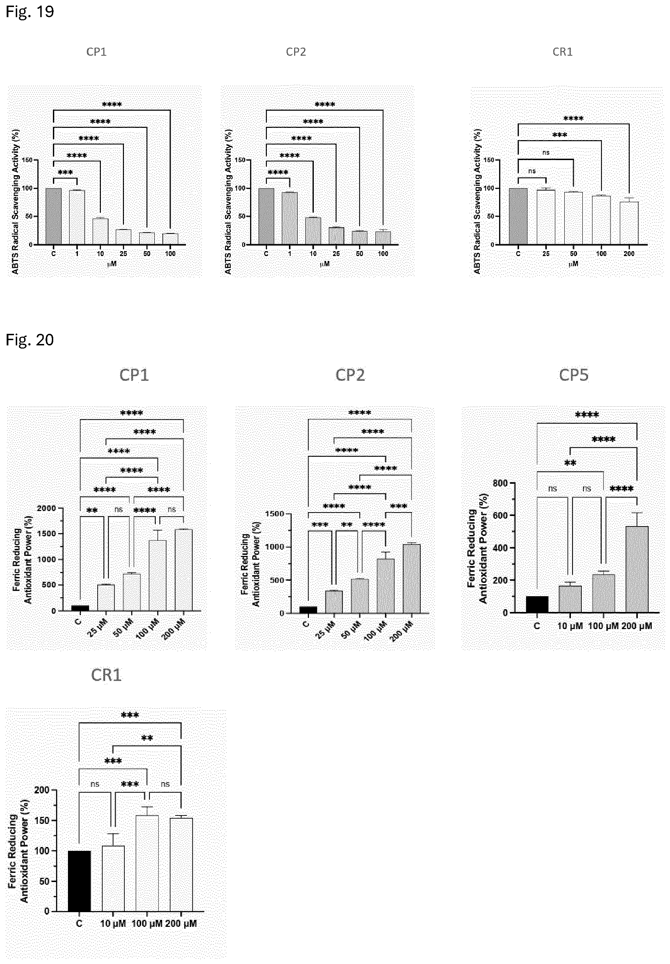

The results are shown in Fig. 19. It can be seen that all three peptides have antioxidant properties, as their ABTS radical scavenging activity is lower than the one of the control C. CP1 and CP2 have stronger antioxidant properties than CR1, as ABTS radical scavenging activity with CP1 and CP2 is lower than with CR1. It is further noted that the ABTS radical scavenging activity decreases with increasing concentration of the peptides.

### Example 20 - Evaluation of antioxidant activity of CP1, CP2, CP5 and CR1 by FRAP

In this example, the antioxidant activity of peptides CP1, CP2, CP5 and CR1 was tested *in vitro* in a FRAP Assay as described above.

The results are shown in Fig. 20. It can be seen that CP1, CP2, CP5 and CR1 have antioxidant properties, as their ferric reducing antioxidant power is higher than the control C. CP1, CP2 and CP5 have stronger antioxidant properties than CR1, as their ferric reducing antioxidant power is higher is higher than with CR1. It is further noted that the ferric reducing antioxidant power increases with increasing concentration of the peptides.

### Example 21 - Evaluation of the protein levels modulation of collagen with the peptides of Table 1

In this example, the modulation of collagen protein levels with the peptides of Table 1 was tested *in vitro* by a Western Blot analysis as described above.

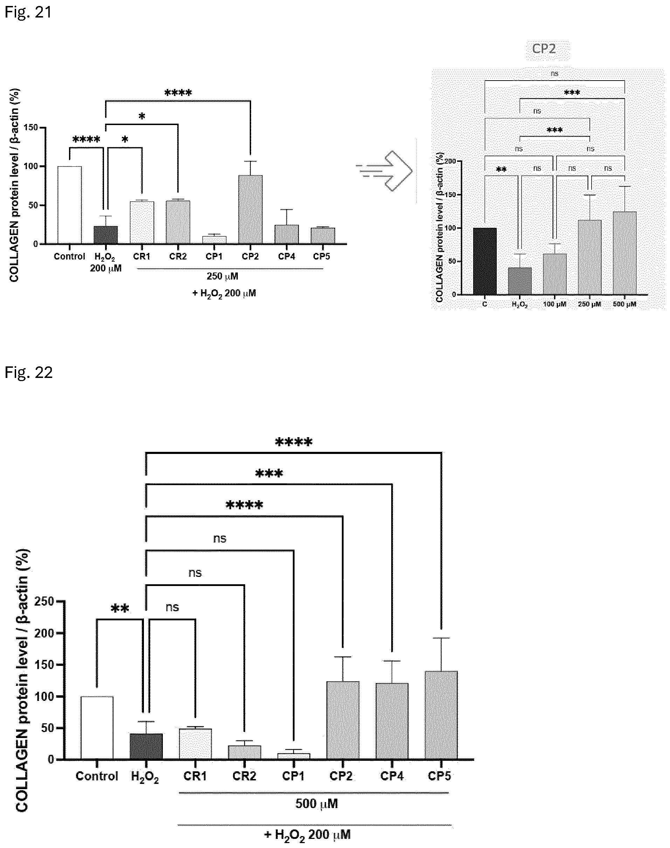

In a first test, screening for the peptide having the highest modulation of collagen protein level at 250 µM was carried out with peptides CR1, CR2, CP1, CP2, CP4, CP5. CP2 resulted to have the highest modulation of collagen protein level. With CP2 additional experiments at different concentrations were carried out. The results are shown in Fig. 21.

Additional experiments with peptides CR1, CR2, CP1, CP2, CP4, CP5 at 500 µM were carried out. The results are shown in Fig. 22. At this concentration CP2, CP4 and CP5 show the highest modulation of collagen protein level.

### Example 22 - Evaluation of the protein levels modulation of elastin with the peptides of Table 1

In this example, the modulation of elastin protein levels with the peptides of Table 1 was tested *in vitro* by a Western Blot analysis as described above.

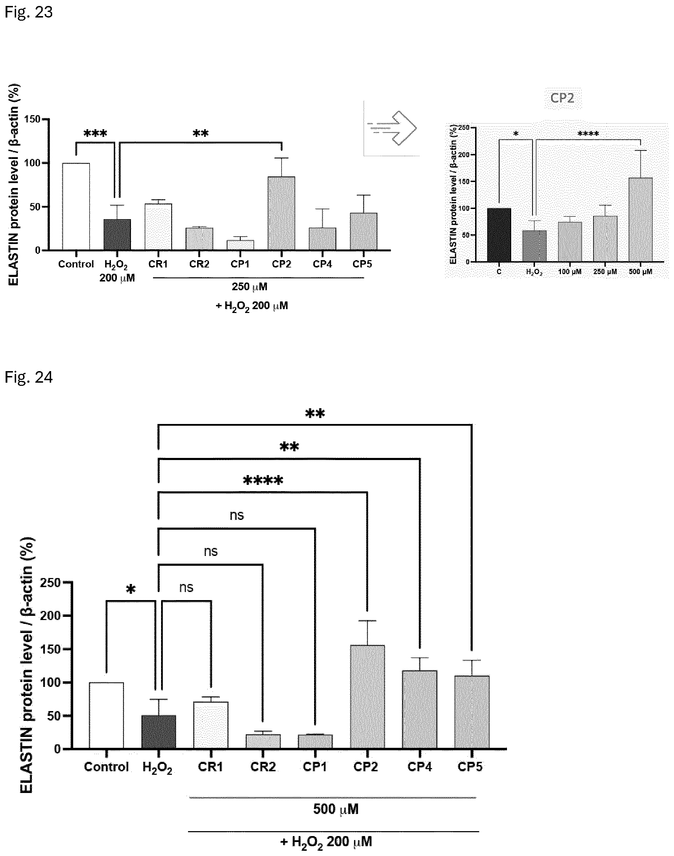

In a first test, screening for the peptide having the highest modulation of elastin protein level at 250 µM was carried out with peptides CR1, CR2, CP1, CP2, CP4, CP5. CP2 resulted to have the highest modulation of elastin protein level. With CP2 additional experiments at different concentrations were carried out. The results are shown in Fig. 23.

Additional experiments with peptides CR1, CR2, CP1, CP2, CP4, CP5 at 500 µM were carried out. The results are shown in Fig. 24. At this concentration CP2, CP4 and CP5 show the highest modulation of elastin protein level, with CP2 showing higher modulation of elastin protein level compared to CP4 and CP5.

### Example 23 - Evaluation of the collagen and elastin secretion with peptides of Table 1

In this example, the secretion of collagen and elastin with the peptides of Table 1 was tested *in vitro* by an ELISA assay as described above. Thereby, ECM stability is evaluated.

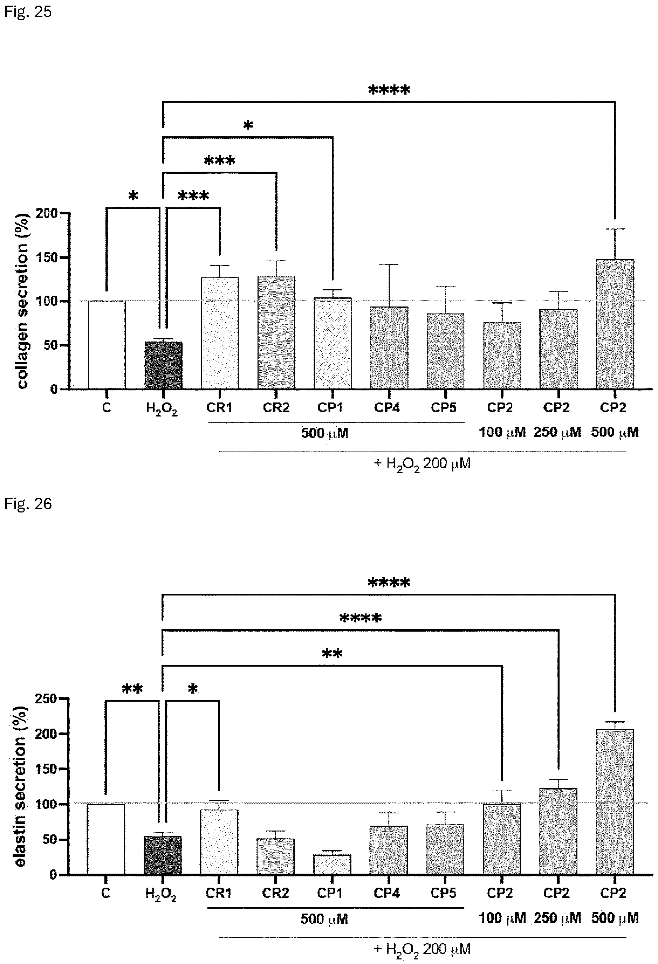

The results are shown in Fig. 25 for collagen and in Fig. 26 for elastin. As regards collagen secretion, CR1, CR2 and CP2 show the strongest stimulation of collagen secretion. As regards elastin secretion, CP2 shows the strongest stimulation of elastin secretion. Even at a concentration of 100 µM, CP2 shows a stimulation higher than for the other tested peptides at a concentration of 500 µM.

## Claims

1. A composition comprising at least a first peptide of a first vegetable origin having skin physiology and/or morphology modifying and/or anti-aging properties.

2. The composition of claim 1, further containing at least a second peptide of a second vegetable origin having skin physiology and/or morphology modifying and/or anti-aging properties.

3. The composition of claim 2, wherein the at least first peptide and the at least second peptide are of different vegetable origin.

4. The composition of anyone of claims 1 to 3, wherein the at least first peptide and the at least second peptide are a peptide originating from a cereal or a fabacea.

5. The composition of anyone of claims 1 to 4, wherein the at least first peptide and/or the at least second peptide is a hydrolyzed peptide derived from, obtained from or contained in a hydrolysate.

6. The composition of claim 5, wherein the at least first peptide or the at least second peptide is a peptide derived from, obtained from or contained in a pea hydrolysate, wherein the at least first peptide or the at least second peptide is one or more of the peptides having the SEQ-ID:1 to SEQ-ID:42.

7. The composition of claim 5 or 6, wherein the at least first peptide or the at least second peptide is a peptide derived from, obtained from or contained in a rice hydrolysate, wherein the at least first peptide or the at least second peptide is one or more of the peptides having the SEQ-ID:43 to SEQ-ID:62.

8. The composition of any one of claims 5 to 7, wherein the at least first peptide of a first vegetable origin is contained in a first hydrolysate and/or the at least second peptide of a second vegetable origin is contained in a second hydrolysate.

9. The composition of any one of claims 1 to 8, wherein the at least first peptide comprises at least one of the three rice peptides with SEQ-:ID: 43, SEQ-ID:45 and SEQ-ID:48 and the at least second peptide comprises at least one of the five pea peptides having SEQ-ID:22, SEQ-ID:23, SEQ-ID:24, SEQ-ID:26, SEQ-ID:31.

10. The composition of any one of claims 1 to 9, wherein the composition further contains at least one of:
- vitamin C,
- biotin,
- zinc in the form of a physiologically compatible zinc compound/zinc salt,
- vitamin E, and
- one or more further components selected from the group consisting of sugars, stabilizers, acidity regulators, preservatives, flavourings and water.

11. The composition of any one of claims 1 to 10, wherein the composition is for oral administration.

12. The composition of any one of claims 1 to 11, wherein the composition is for use as a dietary or food supplement, food for special medical purposes, or for use as a nutraceutical.

13. A composition according to any one of claims 1 to 12, wherein the at least first peptide and/or the at least second peptide are obtained from a method comprising the following steps:
a) preparing a slurry of plant flour or proteins in an aqueous medium,
b) hydrolysing the plant flour or proteins by adding an enzyme to obtain a plant peptide hydrolysate,
c) optionally filtering the plant peptide hydrolysate through a first membrane to give a first permeate and a first concentrate, and
d) optionally filtering the first permeate through a second membrane to give a second permeate and a second concentrate and
e) optionally filtering the first or the second permeate through a third membrane to give a third permeate and a third concentrate.

14. A composition according to claim 13, wherein the first permeate is a low molecular weight fraction comprising at least 11 wt.-% peptides within the 800 to 1900 Da range, and wherein the second permeate is a low molecular weight fraction comprising at least 20 wt.-% peptides within the 800 to 1900 Da range and wherein the third permeate is a low molecular weight fraction comprising a higher weight percentage of peptides within the 800 to 1900 Da range than the first or second permeate.

15. A peptide being one of the peptides having the SEQ-ID:1 to SEQ-ID:62 or a mixture of two or more of the peptides having the SEQ-ID:1 to SEQ-ID:62.
